# EUROPÄISCHE PATENTSCHRIFT

(11) **EP 3 217 909 B1**
(45) Veröffentlichungstag und Bekanntmachung des Hinweises auf die Patenterteilung: **07.05.2025**
(21) Anmeldenummer: 15795146.8
(22) Anmeldetag: 12.11.2015
(51) Int. Cl.: A61B 90/50, A61B 34/00, B25J 13/08, B25J 19/00, A61B 1/00, A61B 90/00

(54) **INTELLIGENTER HALTEARM FÜR DIE KOPFCHIRURGIE MIT BERÜHRUNGSEMPFINDLICHER BEDIENUNG**
INTELLIGENT HOLDING ARM FOR HEAD SURGERY WITH TOUCH-SENSITIVE OPERATION
BRAS DE SUPPORT INTELLIGENT POUR CHIRURGIE DE LA TÊTE AVEC COMMANDES SENSIBLES AU CONTACT

(30) Priorität: 14.11.2014 DE 102014016824; 14.11.2014 DE 102014016823
(43) Veröffentlichungstag der Anmeldung: 20.09.2017
(60) Teilanmeldung: 21205321.9
(73) Patentinhaber: Brainlab Robotics GmbH, 81829 München (DE)
(72) Erfinder: KRINNINGER, Maximilian, 82234 Weßling-Oberpfaffenhofen (DE); NOWATSCHIN, Stephan, 81677 München (DE)
(74) Vertreter: Eisenführ Speiser
(86) Internationale Anmeldenummer: PCT/EP2015/076446
(87) Internationale Veröffentlichungsnummer: WO 2016/075241

(56) Entgegenhaltungen:
- EP-B1- 1 958 587
- EP-B1- 1 958 587
- US-A1- 2002 177 857
- US-A1- 2004 106 916
- US-A1- 2004 106 916
- US-A1- 2004 138 524
- US-A1- 2004 138 524
- US-A1- 2005 209 614
- US-A1- 2005 209 614
- US-A1- 2007 129 846
- US-A1- 2007 129 846
- US-A1- 2010 163 694
- US-A1- 2010 163 694
- US-A1- 2012 143 048
- US-A1- 2012 143 048
- US-A1- 2012 283 747

## Beschreibung

Die Offenbarung betrifft einen Haltearm für medizinische Zwecke, insbesondere zum Halten eines chirurgischen mechatronischen Assistenzsystems und/oder chirurgischen Instruments, mit einem proximalen Ende zum Befestigen des Haltearms an einer Basis und einem distalen Ende zum Aufnehmen eines chirurgischen mechatronischen Assistenzsystems und/oder chirurgischen Instruments; wenigstens einem ersten und einem zweiten Armsegment, wobei das erste Armsegment mit einem ersten Gelenk und das zweite Armsegment mit einem zweiten Gelenk verbunden ist, wobei jedes Gelenk freigebbar und arretierbar ist. Die Offenbarung betrifft ferner ein Verfahren zum Positionieren eines an einem Haltearm gekoppelten chirurgischen mechatronischen Assistenzsystems und/oder chirurgischen Instruments. Weiterhin betrifft die Offenbarung einen Haltearm für medizinische Zwecke, insbesondere zum Halten eines chirurgischen mechatronischen Assistenzsystems mit einem proximalen Ende zum Befestigen des Haltearms an einer Basis und einem distalen Ende zum Aufnehmen eines chirurgischen mechatronischen Assistenzsystems, und zwei oder mehr Armsegmenten und zwei oder mehr Gelenken, mittels derer die Armsegmente gelenkig miteinander verbunden sind, wobei jedes Gelenk mittels einer Bedieneinrichtung freigebbar und arretierbar ist. Ferner betrifft die Offenbarung ein Verfahren zum Steuern eines an einem Haltearm gekoppelten mechatronischen Assistenzsystems, insbesondere unter Verwendung eines derartigen Haltearms.

Haltearme der eingangs genannten Art sind bereits seit längerem bekannt und werden in der Chirurgie insbesondere dazu eingesetzt, einen Operateur von statischer Haltearbeit zu entlasten. Ein derartiger Haltearm wird eingesetzt, um ein mechatronisches Assistenzsystem und/oder ein chirurgisches Instrument zu halten, wie etwa einen Manipulator, ein Endoskop, eine chirurgische Klemme und dergleichen. Insbesondere zum Halten von Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum, ist sehr ermüdend. Aus diesem Grund werden vermehrt Haltearme eingesetzt.

Eine solcher Haltearm ist beispielsweise aus DE 195 26 915 B4 bekannt. Die dort offenbarte Haltevorrichtung für medizinische Zwecke weist ein Anschlussteil und einen Halter für chirurgische Werkzeuge sowie einen zwischen dem Halter und dem Anschlussteil angeordneten Arm auf. Der Arm ist mit dem Halter und dem Anschlussteil oder mit einem benachbarten Arm über ein Gelenk verbunden und mit einer pneumatisch betätigbaren Vorrichtung zur wahlweisen Festlegung und Lösung der Gelenke gekoppelt, wobei diese Vorrichtung die Gelenke unter Einwirkung einer eine Bremskraft auf das Gelenk ausübenden mechanischen Feder festlegt und wobei die Vorrichtung gegen die Kraft dieser Feder pneumatisch in einen das Gelenk freigebenden Zustand überführbar ist. An dem Halter am proximalen Ende des Arms ist ein Betätigungsorgan angeordnet, mittels dessen Hilfe ein Ventil öffenbar ist, sodass die einzelnen Gelenke des Arms verstellt werden können. Bei Loslassen des Betätigungsorgans wird das Ventil wieder geschlossen, sodass die Gelenke festgelegt sind.

Ein ähnlicher Haltearm ist in EP 1 958 587 B1 offenbart. Der dort offenbarte Haltearm weist ebenfalls mehrere Gelenke auf, und zur Betätigung der Gelenke ist ein berührungssensitiver Sensor vorgesehen. Dieser Sensor ist am Haltearm benachbart zum medizinischen Instrument angeordnet, sodass bei Ergreifen des medizinischen Instruments der Operator in Kontakt mit dem berührungssensitiven Sensor kommt, wodurch die Gelenke des Haltearms freigegeben werden.

Sowohl der in DE 195 26 915 B4 als auch in EP 1 958 587 B1 offenbarte Haltearm dient in erster Linie als eine Art Exoskelett für den Operator, sodass sich der Operateur bei der Operation auf diesem Haltearm abstützen kann und bei Ergreifen des medizinischen Instruments oder bei Betätigung des Betätigungsorgans alle Gelenke freigegeben werden, sodass die Pose des Haltearms verändert werden kann.

Aus DE 10 2004 050 714 A1 ist ein weiterer Haltearm bekannt, der zum Halten eines Endoskops ausgebildet ist. Der Arm weist eine Mehrzahl an Gelenken auf, die pneumatisch geschlossen werden können. Der Haltearm ist mit einem Fußschaltventil verbunden. Bei Betätigung des Fußschaltventils gelangt Druckluft in sämtliche Gelenke, wodurch diese freigegeben werden.

Ein weiterer derartiger Haltearm ist in DE 10 2011 004 926 A1 offenbart. Der Haltearm weist mehrere Armsegmente sowie mehrere Gelenke auf, mittels derer die einzelnen Armsegmente miteinander gekoppelt sind. Der Haltearm gemäß DE 10 2011 004 926 A1 weist ferner eine erste Schnittstelle an dem proximalen Ende auf, um den Haltearm mit einer Normschiene eines Operationstisches zu koppeln. Die erste Schnittstelle ist im Wesentlichen gemäß einer Klemme ausgebildet. Darüber hinaus weist der Haltearm eine Schnittstelle am distalen Ende auf, die ebenfalls als Klemme ausgebildet ist und dazu dient, ein Endoskop aufzunehmen. Auch wenn sich dieser Arm grundsätzlich gut zum reinen Halten von Endoskopen eignet, besteht dennoch ein Bedarf, den Einsatzbereich solcher Haltearme flexibler zu gestalten, insbesondere an verschiedene Aufgaben anzupassen. Ferner ist es wünschenswert, die Sicherheit derartiger Haltearme dahingehend zu verbessern, dass ein Verletzungsrisiko eines Patienten während einer Operation, bei der der Haltearm eingesetzt wird, verringert wird.

Nachteilig ist allerdings, dass eine genaue Positionierung des an dem Haltearm angeordneten mechatronischen Assistenzsystems und/oder chirurgischen Instruments mit den vorbekannten Haltearmen nur schwer möglich ist und stark von dem Geschick des Operateurs abhängig ist. Die Positionsgenauigkeit ist allein auf die Fähigkeiten des Operateurs beschränkt, der das distale Ende des Arms im Raum positioniert.

Um dieses Problem zu beheben ist es bekannt, robotisch unterstützte Haltearme zu verwenden, die neben freigeb- und arretierbaren Gelenken auch Motoren in den Gelenken aufweisen, die über ein Terminal steuerbar sind. Mit diesen robotisch angetriebenen Haltearmen ist eine genaue Positionierbarkeit möglich, allerdings ist eine derartige robotische Steuerung sehr aufwändig und bedarf eines ausgiebigen Trainings des Operateurs. Die Bedienung ist komplex und kann daher zu Problemen führen.

Neben den oben genannten Dokumenten ist aus US 2012/0283747 A1 ein chirurgisches System bekannt mit einem Roboterarm, der über eine Steuerung gesteuert werden kann und der einen Haltearm trägt. An dem Haltearm wird dann ein chirurgisches Instrument befestigt, wie zum Beispiel ein Endoskop. Der Haltearm lässt sich nur manuell bedienen und sobald ein Bediener diesen berührt, wird der Roboterarm immobil und unbeweglich. Die Steifigkeit des Haltearms lässt sich einstellen.

US 2012/0143048 A1 offenbart einen Haltearm, der sowohl in einen manuellen Modus als auch in einen robotisch angetriebenen Modus versetzt werden kann. An den drei Gelenken sind jeweils Verriegelungen vorgesehen, mittels derer Gelenke gesperrt oder in ihrer Bewegungsfreiheit eingeschränkt werden können.

Aus US 2002/0177857 A1 ist ferner ein Haltearm bekannt, der an dem distalen Armsegment zwei bezogen auf eine Ebene des durch den Haltearm getragenen Endoskops gegenüberliegende Schalter aufweist, über die alle Gelenke des Haltearms freigegeben werden können. Durch die Lage der Schalter soll ein Bediener immer dieselbe Handposition zum Endoskop haben, wenn er das distale Segment greift.

Aufgabe der vorliegenden Erfindung ist es, einen Haltearm der eingangs genannten Art anzugeben, der einfach insbesondere intuitiv bedienbar ist, und gleichzeitig eine genauere Positionierung des an dem distalen Ende des Arms befestigten mechatronischen Assistenzsystems und/oder chirurgischen Instruments erlaubt.

Diese Aufgabe wird durch einen Haltearm mit den Merkmalen des Anspruchs 1 gelöst, wobei der Haltearm eine Bedieneinrichtung zum Verbringen des Haltearms in eine gewünschte Pose aufweist, wobei die Bedieneinrichtung dazu eingerichtet ist, bei Kontakt zwischen einer Bedienperson und einem der ersten und zweiten Armsegmente das zugehörige Gelenk freizugeben. Erfindungsgemäß ist daher vorgesehen, dass die Bedieneinrichtung dazu eingerichtet ist, bei Kontakt zwischen einer Bedienperson und dem ersten Armsegment das erste Gelenk freizugeben und bei Kontakt zwischen einer Bedienperson und dem zweiten Armsegment das zweite Gelenk freizugeben. Es ist also erfindungsgemäß vorgesehen, dass bei Kontakt einer Bedienperson mit einem entsprechenden Armsegment nur das zugeordnete Gelenk freigegeben wird. Dadurch ist es möglich, intuitiv einzelne Gelenke zu bewegen und den Haltearm so segmentweise zu verstellen und in eine gewünschte Pose zu bringen. Dadurch ist eine genauere Positionierung möglich, da inkrementell jedes Segment separat verstellt werden kann. Es ist ebenso möglich, mehrere Segmente auf einmal zu kontaktieren, sodass mehrere Gelenke gleichzeitig freigegeben werden und so verstellbar sind. Dadurch ist es möglich, den Haltearm auf einfache Art und Weise, insbesondere intuitiv, in eine gewünschte Pose zu überführen. Die hier beschriebenen Verfahren sind nur beispielhaft und sind nicht Teil der vorliegenden Erfindung.

Neben den ersten und zweiten Armsegmenten sind vorzugsweise weitere Armsegmente vorgesehen, die ebenfalls jeweils einem Gelenk zugeordnet sind. Die Armsegmente selbst sind im Wesentlichen starr und vorzugsweise im Wesentlichen stabförmig. Der Begriff "stabförmig" umfasst hier sowohl im Wesentlichen gerade Armsegmente als auch leicht bis stark gekrümmte Armsegmente. Bei einem derartigen Haltearm wechseln sich Armsegmente und Gelenke stets ab, wobei der Haltearm am distalen und am proximalen Ende sowohl mit einem Gelenk als auch mit einem Segment oder einem Anschlusselement enden kann. Mit dem proximalen Ende ist der Haltearm an einer Basis befestigbar. Die Basis kann alternativ fest mit dem Arm gekoppelt sein, oder der Arm ist von der Basis entnehmbar. Die Basis ist in einer Ausführungsform als Operationstisch ausgebildet, und der Haltearm ist mit einem Operationstisch koppelbar. Vorzugsweise ist der Haltearm mit einer an dem Operationstisch vorgesehenen Normschiene koppelbar. Derartige Normschienen sind in der Regel bei Operationstischen vorgesehen, sodass an dem Haltearm zur Kopplung mit der Normschiene eines Operationstisches eine Standardschnittstelle vorgesehen sein kann. Übliche Operationstische sind zudem aus einzelnen Segmenten zusammengesetzt. Zur Kopplung weisen sie Segmente an den Stirnseiten entsprechende Koppelstellen auf, die in der Regel herstellerspezifisch sind. Bevorzugt ist der Haltearm über eine solche Koppelstelle an dem Operationstisch befestigbar. Dazu kann an dem proximalen Ende ein herstellerspezifischer Adapter vorgesehen sein. Alternativ ist die Basis als eine separate Vorrichtung, wie etwa ein Ständer, der auf dem Boden eines Operationssaales aufstellbar ist. In einer weiteren Alternative ist die Basis als eine Halterung ausgebildet, die beispielsweise an einer Wand oder Decke eines Operationssaales befestigbar ist.

Der Haltearm ist vorzugsweise als sogenannter passiver Haltearm ausgebildet und weist daher ausschließlich aktiv gebremste Gelenke auf, jedoch keine angetriebenen Gelenke, wie dies bei robotischen Haltearmen häufig der Fall ist. Jedes Gelenk ist daher nur freigeb- und arretierbar, allerdings nicht antreibbar. Hierdurch ist der Haltearm einfach aufgebaut und bedarf keiner komplexen Steuerung zu seinem Betrieb.

Gemäß einer ersten bevorzugten Ausführungsform der Erfindung weist die Bedieneinrichtung Kontaktmittel auf, die dazu vorgesehen sind, dass ein Bediener mit diesen in Kontakt kommt, wobei ein erstes Kontaktmittel der Bedieneinrichtung an dem ersten Armsegment angeordnet ist und ein zweites Kontaktmittel an dem zweiten Armsegment angeordnet ist. Bei Kontakt mit dem ersten Kontaktmittel wird vorzugsweise das erste Gelenk freigegeben, und bei Kontakt mit dem zweiten Kontaktmittel wird vorzugsweise das zweite Gelenk freigegeben. Die Kontaktmittel dienen dazu, den Kontakt zwischen dem Benutzer und dem Armsegment zu erfassen. Die Kontaktmittel sind vorzugsweise jeweils an einer Oberfläche des entsprechenden Armsegments angeordnet. Die Kontaktmittel können sich über das gesamte Armsegment erstrecken oder nur einen Abschnitt von diesem einnehmen. Vorzugsweise erstreckt sich das Kontaktmittel jeweils in etwa um einen halben Umfang bezogen auf eine Zentralachse eines Armsegments. Dadurch ist das Kontaktmittel in jeder Pose des Haltearms leicht zugänglich, und ein Bediener kann leicht mit diesem in Kontakt kommen.

Gemäß einer weiteren bevorzugten Ausführungsform weist jedes Kontaktmittel zwei an dem Armsegment im Wesentlichen gegenüberliegend angeordnete Kontaktmittelelemente auf. Gemäß diesem Ausführungsbeispiel ist es bevorzugt, dass das zugeordnete Gelenk nur bei Kontakt von den beiden Kontaktmittelelementen freigegeben wird. Das Kontaktmittel besteht vorzugsweise aus den zwei Kontaktmittelelementen, sodass ein Kontakt mit dem Kontaktmittel nur dann vorliegt, wenn beide Kontaktmittelelemente von dem Bediener kontaktiert werden. Indem die beiden Kontaktmittelelemente im Wesentlichen gegenüberliegend, vorzugsweise bezogen auf eine Ebene, die eine Zentralachse des Armsegments enthält, angeordnet sind, ist es möglich, einen unbeabsichtigten Kontakt, beispielsweise durch den Arm eines Bedieners, von einem beabsichtigten Kontakt, einem konkreten Greifen des Armsegments, zu unterscheiden, folglich wird gemäß diesem Ausführungsbeispiel das Gelenk nur bei einem Ergreifen des Armsegments, insbesondere durch die Hand des Bedieners, bei dem die beiden gegenüberliegenden Seiten des Armsegments kontaktiert werden, freigegeben. Zur Bedienung des Haltearms und zum Verbringen des Haltearms in eine gewünschte Pose mittels der Bedieneinrichtung ist folglich das Armsegment durch den Bediener so zu ergreifen, dass er in Kontakt mit den beiden Kontaktmittelelementen des Kontaktmittels kommt, woraufhin dann durch die Bedieneinrichtung das zugeordnete Gelenk freigegeben wird und das Armsegment bewegt werden kann.

Gemäß einer bevorzugten Weiterbildung sind die Kontaktmittelelemente als Taster ausgebildet. Taster sind besonders einfache Elemente, die sowohl optisch durch den Bediener erfasst werden können, als auch unmittelbar ein taktiles Feedback beim Drücken des Tasters bieten. Ein solcher Taster kann beispielsweise als einfacher Schließer eines elektrischen Kreislaufs oder als kapazitiver Schalter ausgebildet sein. Solange gemäß diesem Ausführungsbeispiel beide Taster gedrückt sind, wird das dem entsprechenden Armsegment zugeordnete Gelenk freigegeben; sobald der Bediener die beiden oder auch nur einen der beiden Taster loslässt, wird das Gelenk durch die Bedieneinrichtung wieder arretiert.

Gemäß einer bevorzugten alternativen Ausführungsform sind die Kontaktmittelelemente als berührungsempfindliche Sensoren ausgebildet. Die Sensoren sind vorzugsweise im Wesentlichen flächig ausgebildet und erstrecken sich über einen wesentlichen Abschnitt der Oberfläche des entsprechenden Armsegments. Vorzugsweise sind die Sensoren als druckempfindliche Sensoren, kapazitive Sensoren, wärmeempfindliche Sensoren und/oder optische Sensoren ausgebildet. Derartige Sensoren haben den Vorteil, dass sie eine größere Fläche abdecken können, der Bediener also das Armsegment nicht so genau kontaktieren muss, sondern es ausreicht, dass der Bediener das Armsegment im Wesentlichen umgreift und so mit dem Sensor beziehungsweise den Sensoren in Kontakt kommt.

In einer weiteren bevorzugten Ausgestaltung ist die Bedieneinrichtung dazu ausgebildet, in Abhängigkeit der Intensität des Kontakts das zugeordnete Gelenk freizugeben. Intensität bezieht sich hier auf einen Druck und/oder eine Kraft, der bzw. die durch den Bediener aufgebracht wird. So ist es möglich, dass der Bediener durch die Kraft, die er beim Zugreifen aufbringt, einen Grad der Freigabe steuert. So ist es denkbar und bevorzugt, dass das zugeordnete Gelenk bei einer geringen Intensität des Kontakts nur teilweise freigegeben wird, sodass das Armsegment nur langsam und gegen einen Widerstand bewegbar ist. Bei einer hohen Intensität und demzufolge einem festen Zugreifen wird das Gelenk dann vollständig geöffnet, sodass das Armsegment im Wesentlichen widerstandsfrei beweglich ist. Ein teilweises Freigeben kann auch durch ein intermittierendes Freigeben in verschiedenen Frequenzen realisiert werden.

Gemäß einer weiteren bevorzugten Ausführungsform weist die Bedieneinrichtung Anzeigemittel zum Anzeigen eines Kontakts zwischen einem Bediener und dem Armsegment auf. Bevorzugt sind die Anzeigemittel dazu eingerichtet, ein taktiles, visuelles und/oder ein Autosignal auszugeben. Gemäß dieser Ausführungsform wird also bei Kontakt zwischen dem Bediener und dem Armsegment nicht nur das zugeordnete Gelenk freigegeben, sondern gleichzeitig als Rückmeldung für den Bediener ein Signal ausgegeben, insbesondere ein taktiles, visuelles und und/oder ein Audiosignal. Beispielsweise ist es bevorzugt, dass Kontaktmittelelemente von Kontaktmitteln ein Leuchtmittel, wie etwa eine LED oder dergleichen, aufweisen, welche bei Kontakt zwischen dem Bediener und dem Kontaktmittelelement aufleuchten. Ein taktiles Signal umfasst beispielsweise eine Vibration. Ein Audiosignal kann einen einfachen Ton oder auch eine Sprachausgabe wie etwa "Gelenk freigegeben" umfassen.

Gemäß einer weiteren bevorzugten Ausgestaltung weist die Bedieneinrichtung ferner einen Schalter zum Freigeben aller Gelenke auf. Ein derartiger Schalter, mittels dessen alle Gelenke freigebbar sind, kann als Masterschalter bezeichnet werden. Er kann ausgebildet sein, wie beispielsweise in EP 1 958 587 B1 offenbart, also als Schalter am distalen Ende des Haltearms, oder wie in DE 195 26 915 B4 offenbart, als Fußschalter, der beabstandet zum Haltearm angeordnet ist. Ein derartiger Schalter, der als Masterschalter wirkt, ist vorteilhaft, um eine Grobpositionierung des Haltearms, beispielsweise zu Beginn eines Operationsvorgangs, vorzunehmen. Dann ist es möglich, alle Gelenke gleichzeitig freizugeben und den Haltearm in eine grobe Vorpositionierung zu bringen und anschließend durch Loslassen des Schalters sämtliche Gelenke wieder zu sperren und nur durch Kontakt mit einzelnen Armsegmenten diese entsprechend zu verstellen, um eine gewünschte Pose zu erreichen. Bevorzugt ist vorgesehen, dass der Schalter zwei unterschiedliche Schaltstellungen aufweist, wobei die Gelenke in einer ersten Schaltstellung derart freigegeben werden, dass ein Tool Center Point eines an dem Haltearm befestigten mechatronischen Assistenzsystems und/oder chirurgischen Instruments, rotierbar ist, und in einer zweiten Schaltstellung des Schalters die Gelenke derart freigegeben werden, dass der Tool Center Point translatorisch in drei Raumrichtungen bewegbar ist. Auf diese Weise lässt sich eine besonders bevorzugte Grobpositionierung des Haltearms erreichen.

In einer bevorzugten Ausgestaltung der Erfindung ist vorgesehen, dass das erste Gelenk an einem proximalen Ende des ersten Armsegments angeordnet ist und dass das zweite Gelenk an einem proximalen Ende des zweiten Armsegments angeordnet ist. Jedes Armsegment hat ein proximales und ein distales Ende, wobei das proximale Ende des Armsegments dasjenige Ende ist, welches in Armrichtung proximal zum proximalen Ende des Haltearms liegt, und das distale Ende des Armsegments dasjenige Endet ist, das entlang des Haltearms zum distalen Ende hin ausgerichtet ist. Gemäß dieser Ausführungsform befindet sich das erste Gelenk folglich zwischen dem ersten Armsegment und dem proximalen Ende des Haltearms, sodass bei Kontakt mit dem ersten Armsegment das erste Armsegment bewegbar ist. Entsprechendes gilt für das zweite Armsegment und das zweite Gelenk. Durch diese Ausgestaltung wird eine besonders intuitive Bedienbarkeit des Haltearms erreicht, da stets dasjenige Armsegment bewegbar ist, welches der Bediener kontaktiert.

In einer bevorzugten Alternative ist vorgesehen, dass die Bedieneinrichtung bei Kontakt zwischen der Bedienperson mit sowohl dem ersten und als auch dem zweiten Armsegment alle zwischen diesen Armsegmenten angeordneten Gelenke freigibt. Ergreift die Bedienperson bei einem Haltearm gemäß dieser Ausführungsform zwei Armsegmente, die benachbart oder entfernt voneinander sind, werden folglich alle zwischen diesen Armsegmenten liegenden Gelenke (ein oder mehrere Gelenke) freigegeben. Der Bediener ist also in der Lage bei Ergreifen von zwei Armsegmenten das distale Armsegment im Wesentlichen frei zu dem proximalen Armsegment der beiden ergriffenen zu bewegen. Er kann auch alle Gelenke des Haltearms freigeben, nämlich dann wenn der sowohl das distale als auch das proximale Armsegment ergreift. Hierdurch ist eine einfache und intuitive Freigabe von einem oder mehreren Gelenken erreicht.

Gemäß einer weiteren bevorzugten Ausführungsform der Erfindung weisen die Gelenke Bremsen auf, mittels derer die Gelenke freigebbar und arretierbar sind. Der Haltearm ist vorzugsweise als passiver Haltearm ausgebildet. Die Bremsen dienen dazu, eine Bewegung der Armsegmente relativ zueinander, also eine Bewegung der Gelenke zu bremsen beziehungsweise zu verhindern. Sind die Bremsen gelöst, sind die Gelenke freigegeben.

Vorzugsweise sind die Bremsen in einem Ruhezustand derart vorgespannt, dass die Gelenke arretiert sind. Dadurch befindet sich der Haltearm, wenn dieser in einem Ruhezustand ist, in einem arretierten Zustand, wodurch die Sicherheit des Haltearms bei der Verwendung im medizinischen Bereich verbessert ist. Kommt es beispielsweise aufgrund einer Störung zu einem Ausfall einer Energieversorgung, befindet sich der Haltearm in einem arretierten Zustand, und die Pose des Haltearms bleibt bestehen. Ferner ist durch eine derartige Gestaltung der Vorspannung ein Energiebedarf des Haltearms reduziert.

Besonders bevorzugt sind die Bremsen als elektromagnetische Bremsen ausgebildet und weisen jeweils einen Permanentmagneten auf, der die Bremsen im unbestromten Zustand in den arretierten Zustand vorspannt. Dies ist eine besonders zweckdienliche Gestaltung der Bremsen. Zum Freigeben sind die Bremsen zu bestromen, sodass ein Bremselement entgegen der Kraft des Permanentmagneten gelöst wird. Bei Ausfall einer Stromversorgung schließt die Bremse aufgrund des Permanentmagneten wieder, sodass das Gelenk dann im arretierten Zustand ist. Elektromagnetische Bremsen haben den Vorteil, dass sie im Verhältnis zu ihrem Gewicht eine hohe Haltekraft bzw. ein hohes Haltemoment ausüben können, verglichen mit Federdruckbremen.

Gemäß einer weiteren bevorzugten Ausgestaltung weist der Haltearm sechs Freiheitsgrade auf. Besonders bevorzugt weist der Haltearm sieben Freiheitsgrade auf. Während sechs Freiheitsgrade ausreichen, um jeden Punkt im Raum zu erreichen, ist es bei sieben Freiheitsgraden möglich, jeden Punkt mit verschiedenen Posen zu erreichen, sodass der Haltearm stets so ausrichtbar ist, dass beispielsweise das Operationsfeld leicht zugänglich ist. Daher ist es besonders bevorzugt, dass der Haltearm sieben Freiheitsgrade aufweist.

Gemäß einer bevorzugten Ausführungsform weist der Haltearm sieben Armsegmente und sieben Gelenke auf, wobei jedem Armsegment ein Gelenk zugeordnet ist. Jedes Gelenk weist gemäß diesem Ausführungsbeispiel vorzugsweise einen Freiheitsgrad auf, sodass der Haltearm insgesamt sieben Freiheitsgrade aufweist. Es ist auch möglich, dass jedes Gelenk zwei oder mehr Freiheitsgrade hat, wobei Gelenke mit einem Freiheitsgrad aufgrund ihrer Stabilität bevorzugt sind. Vorzugsweise sind sämtliche Gelenke als rotatorische Gelenke ausgebildet. Vorzugsweise sind einige der Gelenke als rotatorische Gelenke und einige als translatorische Gelenke ausgebildet. Vorzugsweise sind die Gelenke, wenn diese sämtlich rotatorisch ausgebildet sind, derart in dem Haltearm angeordnet, dass Achsen von entlang des Haltearms aufeinanderfolgenden Gelenken, vom proximalen zum distalen Ende des Haltearms hin, jeweils senkrecht aufeinander stehen.

Gemäß einer weiteren bevorzugten Ausgestaltung weist der Haltearm eine Gewichtskompensationseinrichtung zum wenigstens teilweisen Abstützen des Gewichts eines oder mehrerer Armsegmente des Haltearms auf, wenn ein oder mehrere Gelenke freigegeben sind. Ist beispielsweise ein Gelenk nahe dem proximalen Ende des Haltearms freigegeben, muss der Bediener händisch das gesamte Gewicht des restlichen Haltearms bis zum distalen Ende halten. Um ein Absacken des Haltearms nach der Freigabe zu verhindern, ist vorzugsweise eine Gewichtskompensationseinrichtung vorgesehen, die entsprechend das Gewicht wenigstens teilweise kompensiert. Dadurch muss nach Freigabe der Bediener nur ein geringeres Gewicht abstützen, wodurch die Handhabung weiter vereinfacht ist.

In einer bevorzugten Weiterbildung weist die Gewichtskompensationseinrichtung ein Gasdruckfederelement auf, welches mit wenigstens zwei Armsegmenten gekoppelt ist. Ein derartiges Gasdruckfederelement ist eine einfache Möglichkeit, eine Gewichtskompensationseinrichtung auszubilden. Vorzugsweise ist das Gasdruckfederelement mit den zwei am proximalen Ende des Haltearms angeordneten Armsegmenten gekoppelt. Vorzugsweise sind diese beiden Armsegmente mit einem Gelenk verbunden, dessen Schwenkachse senkrecht zu einer Längserstreckung der Armsegmente ausgerichtet ist. Dadurch ist eine besonders zweckdienliche Anordnung des Gasdruckfederelements erreicht, und an dem Haltearm angreifende Kräfte sind vorteilhaft abstützbar.

Weiterhin ist bevorzugt, dass die Gewichtskompensationseinrichtung wenigstens ein Federelement, insbesondere eine ein Drehmoment aufbringende Feder, wie etwa eine Spiralfeder oder eine Drehstabfeder, in wenigstens einem Gelenk aufweist. Zusätzlich oder alternativ zu dem Gasdruckfederelement sind demnach ein oder mehrere Federelemente in den Gelenken vorgesehen. Beispielsweise ist eine Spiralfeder in einem rotatorischen Gelenk vorgesehen, und bringt ein Moment derart auf das Gelenk auf, dass ein Abschnitt des Haltearms abgestützt wird, wenn das Gelenk freigegeben ist. Derartige Spiralfedern sind eine weitere einfache Möglichkeit, eine Gewichtskompensation vorzusehen.

In einer weiteren bevorzugten Ausgestaltung ist ferner vorgesehen, dass an den Armsegmenten Ausrichtungsindikatoren angeordnet sind, die eine Grundpose des Haltearms angeben. Dies ist besonders bevorzugt, wenn zur Gewichtskompensation Federelemente in den Gelenken vorgesehen sind. Dadurch, dass Ausrichtungsindikatoren vorgesehen sind, kann ein Benutzer stets auf einfache Art und Weise erkennen, ob sich ein Haltearm in einer Grundpose oder in einer dazu abweichenden Pose befindet. So kann verhindert werden, dass beim Freigeben eines Gelenks eine Gewichtskompensationseinrichtung dazu führt, dass die Gewichtskompensationseinrichtung den Haltearm in Bewegungsrichtung vorspannt und so nicht das Gewicht kompensiert, sondern dieses zusätzlich verstärkt. Als eine Alternative ist beispielsweise eine Innenseite, beziehungsweise eine Seite des Haltearms, die im Normalbetrieb zum Operationsfeld hin orientiert ist, in einer ersten Farbe gestaltet, während eine nach außen gerichtete, beziehungsweise vom Operationsfeld weg orientierte Seite des Haltearms in einer zweiten Farbe gestaltet ist. Alternativ sind Vorsprünge, Beschriftungen oder dergleichen vorgesehen, die als Ausrichtungsindikatoren dienen.

Weiterhin ist bevorzugt, dass innerhalb der Armsegmente wenigstens ein Kabelkanal zum Durchführen von Kabeln von dem proximalen zum distalen Ende des Haltearms vorgesehen ist. Es ist bevorzugt, derartige Kabel, beispielsweise für elektrische Spannungsversorgung, Druckluftversorgung, Lichtübertragung und dergleichen innerhalb des Haltearms zu führen, sodass außerhalb von diesem keine Verkabelung verläuft, die im Betrieb Komplikationen hervorrufen könnte.

In einer weiteren bevorzugten Ausführungsform weist das erste Armsegment, bezogen auf das proximale Ende des Haltearms, erste mechanische Kopplungsmittel zum lösbaren Koppeln des Haltearms mit zweiten korrespondierenden Kopplungsmitteln eines Operationstisches auf. Dadurch ist der Haltearm auf einfache Art und Weise mit einem Operationstisch, insbesondere der Normschiene, koppelbar.

Weiterhin ist bevorzugt, dass das letzte Armsegment am distalen Ende des Haltearms eine mechatronische Schnittstelle zum Koppeln des chirurgischen mechatronischen Assistenzsystems und/oder chirurgischen Instruments mit dem Haltearm aufweist. Eine derartige mechatronische Schnittstelle weist vorzugsweise mechanische Kopplungsmittel zum mechanischen Halten des Assistenzsystems und/oder chirurgischen Instruments auf, und andererseits elektronische Schnittstellen zum Übertragen von elektrischer Energie und/oder Daten beziehungsweise Signalen an das mechatronische Assistenzsystem.

Weiterhin wird die Aufgabe bei einem Haltearm der eingangs genannten Art, insbesondere einem Haltearm für medizinische Zwecke, insbesondere zum Halten eines chirurgischen mechatronischen Assistenzsystems mit einem proximalen Ende zum Befestigen des Haltearms an einer Basis und einem distalen Ende zum Aufnehmen eines chirurgischen mechatronischen Assistenzsystems, und zwei oder mehr Armsegmenten und zwei oder mehr Gelenken, mittels derer die Armsegmente gelenkig miteinander verbunden sind, wobei jedes Gelenk mittels einer Bedieneinrichtung freigebbar und arretierbar ist, gelöst durch eine erste Schnittstelle an dem proximalen Ende zum Verbinden des Haltearms mit einer Energiequelle sowie mit einer externen Steuereinheit zum Übertragen von Signalen an den und von dem Haltearm; eine zweite Schnittstelle an dem distalen Ende zum Koppeln des Haltearms mit dem Assistenzsystem zum Steuern des Assistenzsystems; und eine Übertragungseinrichtung, welche innerhalb des Haltearms angeordnet ist und die erste Schnittstelle mit der zweiten Schnittstelle zum Übertragen von Energie und Signalen zwischen den Schnittstellen verbindet.

Als Assistenzsysteme in dem erfindungsgemäßen Sinn werden jegliche Art von mechatronischen Manipulatoren, die in der Chirurgie eingesetzt werden, verstanden, so wie insbesondere Endoskope, Exoskope, Laparoskope, Trokare und dergleichen. Die zweite Schnittstelle am distalen Ende des Haltearms ist dazu ausgebildet, sowohl mechanisch mit dem Assistenzsystem zu koppeln, um dieses in einer definierten Position zum Haltearm zu halten, als auch die notwendigen weiteren Anschlüsse, wie insbesondere einen Anschluss für elektrische Energie und einen Anschluss zum Übertragen von Signalen, insbesondere Stellsignalen, bereitzustellen. Innerhalb des Haltearms ist eine Übertragungseinrichtung ausgebildet, die vorzugsweise ein Bussystem aufweist. Ferner weist die Übertragungseinrichtung Mittel zum Übertragen von elektrischer Energie auf. Dadurch sind sämtliche Kabel, die zur Übertragung von elektrischer Energie und/oder Daten von der ersten Schnittstelle zur zweiten Schnittstelle erforderlich sind, im Inneren des Haltearms angeordnet und dadurch während des Betriebs des Haltearms geschützt. An der ersten Schnittstelle sind weiterhin Mittel vorgesehen, um den Haltearm mit einer Energiequelle und einer externen Steuereinheit, wie etwa einem Computer und/oder einem OP-System, zu koppeln. Hierdurch ist der Einsatzbereich des Haltearms vergrößert, und dieser kann flexibel für verschiedene Assistenzsysteme eingesetzt werden. Gleichzeitig ist die Sicherheit verbessert, da das Anbringen zusätzlicher Kabel oder dergleichen nicht erforderlich ist, sondern das Assistenzsystem ist lediglich mit der zweiten Schnittstelle am distalen Ende zu koppeln und der Haltearm selbst wiederum über die erste Schnittstelle am proximalen Ende mit einer Energiequelle und einer externen Steuereinheit koppelbar.

Besonders bevorzugt weist die erste Schnittstelle einen Anschluss für einen externen Akkumulator auf. Dadurch ist der Haltearm unabhängig von einer stationären Stromversorgung, und Kabel können weiterhin vermieden werden. Auch ist der Haltearm so im Falle eines Stromausfalls weiterhin betriebsbereit, wodurch die Sicherheit verbessert ist. Die erste Schnittstelle weist vorzugsweise einen Anschluss zur Anbindung des Haltearms an eine Navigationseinrichtung, insbesondere eine OP-Navigationseinrichtung, auf. In modernen Operationssälen werden meist mehrere robotische Systeme eingesetzt. Indem der Haltearm eine Anbindung zu einem solchen Navigationssystem aufweist, ist es möglich, dass dieser Positionsdaten übergibt und empfängt, und so kann eine Kollision mit anderen robotischen Systemen vermieden werden. Weiterhin weist die erste Schnittstelle vorzugsweise einen Bluetooth^{®}-, einen USB-, einen RS-232, und/oder einen optischen Anschluss auf. Mittels eines Bluetooth^{®}-Anschlusses ist es beispielsweise möglich, Signale an den Haltearm zu übertragen und diese über die Übertragungseinrichtung an der zweiten Schnittstelle bereitzustellen, wo diese dann an ein chirurgisches mechatronisches Assistenzsystem, wie etwa einen Manipulator, übergeben werden. Das gleiche gilt für USB- und RS-232-Schnittstellen. USB- und RS-232-Schnittstellen eignen sich besonders, um herkömmliche PCs oder OP-Systeme an den Haltearm anzuschließen.

In einer weiteren bevorzugten Ausgestaltung weist die erste Schnittstelle einen Anschluss für ein Röntgengerät, ein Ultraschallgerät oder einen medizinischen Laser auf, und die Übertragungseinrichtung ist dazu ausgebildet, Röntgenstrahlen, Ultraschallsignale und/oder einen medizinischen Laserstrahl zu übertragen, und die zweite Schnittstelle ist dazu eingerichtet, Röntgenstrahlen, Ultraschallsignale und/oder Laserstrahlen auf ein Operationsfeld abzugeben, oder an ein chirurgisches mechatronisches Assistenzsystem bereitzustellen. Hierdurch ist es weiterhin möglich, den Haltearm flexibel einzusetzen, und eine Verkabelung im Operationsbereich kann weitgehend vermieden werden. Röntgengeräte, Ultraschallgeräte und medizinische Laser können distal zum Patienten angeordnet werden und mittels des Haltearms zum Operationsfeld hin übertragen und bereitgestellt werden.

In einer weiteren bevorzugten Ausführungsform weist der Haltearm in wenigstens einem Gelenk einen Lagesensor auf, zum Erfassen einer Stellung des Gelenks. Vorzugsweise ist in jedem Gelenk ein Lagesensor zum Erfassen der Stellung eines Gelenks angeordnet. Ein derartiger Lagesensor kann beispielsweise als kapazitiver Wegaufnehmersensor ausgebildet sein, der mechanisch einen Bewegungsweg des Gelenks aufnimmt, und so eine Winkelstellung bestimmt, oder als Beschleunigungssensor, der eine Bewegung des Gelenks im Raum bestimmt. Zusätzlich oder alternativ sind Bewegungssensoren in den Armsegmenten vorgesehen, sodass die Lage der Armsegmente im Raum bestimmbar ist. Hierdurch ist eine Pose des Haltearms bestimmbar, welche wiederum über die erste und/oder zweite Schnittstelle an das Assistenzsystem und/oder an die externe Steuereinheit bereitgestellt werden kann. Dies ist insbesondere vorteilhaft, wenn ein OP-Navigationssystem verwendet wird und dieses die Informationen über die Pose des Haltearms verwendet, um eine Navigation zu koordinieren. Weiterhin ist über die Pose des Haltearms ermittelbar, ob eine Kollision mit weiteren Geräten oder dem Haltearm selbst droht. Hierdurch ist die Sicherheit des Haltearms weiter verbessert.

Wird ein Beschleunigungssensor als Lagesensor verwendet, ist es auch möglich, eine Bewegung des Haltearms insgesamt, ohne Veränderung der Pose zu detektieren. Wird beispielsweise der Operationstisch während der Operation bewegt, kann der Haltearm diese Bewegung erfassen. Es kann vorgesehen sein, dass der Haltearm bei einer bestimmten Neigung des Operationstisches, beispielsweise ab 15 Grad, dazu eingerichtet ist, ein Warnsignal auszugeben. Wird eine Neigung des Operationstisches zu steil eingestellt, ist es möglich, dass ein Patient auf dem Operationstisch verrutscht, wodurch Verletzungen hervorgerufen werden können. Ist beispielsweise an der distalen Schnittstelle des Haltearms ein Endoskop angeordnet, welches beispielsweise in die Nase eines Patienten eingeführt ist, und wird dann die Neigung des Tisches verstellt, so detektiert der Haltearm mittels der Bewegungssensoren einerseits die Neigung des Tisches, andererseits aufgrund von Drehmomentsensoren in den Gelenken auch eine Belastungsänderung an dem Endoskop, was ebenfalls ein Verrutschen des Patienten auf dem Operationstisch andeuten kann. Vorzugsweise ist der Haltearm dazu eingerichtet, bei Überschreiten vorbestimmter Grenzwerte ein Signal, beispielsweise einen Signalton auszugeben. Es kann auch vorgesehen sein, dass der Haltearm ein Signal, beispielsweise an ein OP-System oder direkt an einen Operationstisch, ausgibt wenn der Haltearm mittels der proximalen Schnittstelle an ein OP-System oder den Operationstisch gekoppelt ist, derart, dass eine Bewegung des Tisches blockiert wird, wenn sich ein an der distalen Schnittstelle angeordnetes Endoskop in dem Situs befindet. Ferner kann vorgesehen sein, dass nach einer detektierten Patientenbewegung oder einer Tischbewegung relativ zu dem Haltearm, eine zuvor angefahrene Position eines Assistenzsystems, insbesondere eines Endoskops, relativ zum Patienten nun nicht mehr angefahren werden darf. Auch dadurch kann eine Verletzung vermieden werden.

Ist zusätzlich ein OP-Navigationssystem vorhanden, ist es mittels des erfindungsgemäßen Haltearms zudem möglich, auch nicht navigierte Instrumente, die keine dem Navigationssystem zugeordneten Lokalisatoren aufweisen, über den Haltearm in das Navigationssystem zu integrieren. Mittels des Haltearms ist es möglich, die Position eines nicht navigierten Instruments, welches an der distalen Schnittstelle des Haltearms angeordnet ist, zu bestimmen. Dazu ist an dem Haltearm oder an der Basis, mit der der Haltearm gekoppelt ist, ein Lokalisator angeordnet, sodass die Position des Haltearms in dem Navigationssystem bekannt ist. Der Haltearm kann über die proximale Schnittstelle Daten, die die Lage des Instruments repräsentieren, an das Navigationssystem übergeben, dieses kann die Daten verarbeiten und so das nicht navigierte Instrument in die Navigation integrieren. Neben Beschleunigungssensoren sind auch andere Positions- und Gyroskopsensoren bevorzugt. Indem Instrumente, die selber über keine Sensorik oder dergleichen verfügen (wie etwa chirurgische Klemmen und dergleichen) mittels des Haltearms gehalten werden, ist deren Position relativ zum Tisch bekannt. Häufig bestehen Operationstische aus einzelnen Segmenten, die manuell verstellt werden können. Beispielsweise kann eine Kopfplatte angehoben, geneigt oder verschoben werden. Hierdurch ändert sich häufig die relative Lage des Patienten zu Instrumenten, wodurch Verletzungen hervorgerufen werden können. Indem der Haltearm Lagesensoren aufweist, kann dieser eine Bewegung des Operationstisches oder Segmente des Operationstisches detektieren und so vor einer Relativbewegung des Patienten zum Assistenzsystem (beispielsweise der chirurgischen Klemme) warnen, indem bei einer derartigen detektieren Relativbewegung ein Signal ausgegeben wird. Es ist auch möglich, dass der Haltearm über die proximale Schnittstelle Daten über seine Pose an weitere Systeme wie etwa C-Bögen (beispielsweise das System Artis zeego der Firma Siemens AG, Erlangen, Deutschland), wodurch Kollisionen vermieden werden können.

In einer weiteren bevorzugten Ausgestaltung ist in wenigstens einem Gelenk ein Drehmomentsensor zum Erfassen eines auf das Gelenk wirkenden Drehmoments angeordnet. Vorzugsweise ist in allen Gelenken ein derartiger Drehmomentsensor angeordnet. Über das Erfassen von auf die Gelenke wirkenden Drehmomenten ist es möglich, eine Kraft, die am distalen Ende des Haltearms wirkt, zu bestimmen. Dadurch ist einerseits ein Gewicht eines Assistenzsystems bestimmbar, welches an dem distalen Ende gekoppelt ist. Andererseits sind auch bei der Verwendung des Haltearms auf diesen wirkende Kräfte bestimmbar. So ist es beispielsweise denkbar, dass an der zweiten Schnittstelle ein Endoskop angeordnet ist. Beim Handhaben des Endoskops, beispielsweise Einführen des Endoskops in eine Körperöffnung eines Patienten, kann so ein Widerstand bestimmt werden, auf den das Endoskop stößt. Dadurch ist erkennbar, ob eine Verletzung des Patienten droht. Hierdurch ist die Sicherheit weiter verbessert. Vorzugsweise werden die erfassten Drehmomentdaten an der ersten und/oder zweiten Schnittstelle bereitgestellt. Dadurch können die Drehmomentdaten an der externen Steuereinheit verarbeitet werden, und diese kann, beispielsweise im oben genannten Fall der Kollision eines Endoskops mit einem Widerstand im Körper eines Patienten, ein Warnsignal oder dergleichen ausgeben.

Mittels der Drehmomentsensoren ist ferner ein Gewicht eines an der distalen Schnittstelle angeordneten Assistenzsystems bestimmbar. Der Haltearm ist vorzugsweise so ausgelegt, dass er Posen, in denen ein bestimmter Drehmoment-Schwellwert eines Gelenks, aufgrund eines Gewichts des Assistenzsystems überschritten werden würde, blockiert. Wird beispielsweise ein relativ schweres Endoskop an dem distalen Ende des Haltearms angeordnet, wird eine Pose, bei der der Haltearm sehr weit von der Basis auskragt, und demnach ein sehr hohes Moment in einem proximalen Drehgelenk wirkt, blockiert. Ein Operateur kann den Haltearm nicht in eine solche Pose bewegen, da zuvor Bremsen in Gelenken das Verbringen des Haltearms in eine derartige Pose verhindern. Dadurch ist die Sicherheit des Haltearms weiter verbessert. Es ist auch denkbar, dass das Gewicht des Assistenzsystems nicht von dem Haltearm bestimmt wird, sondern das Assistenzsystem sein eigenes Gewicht an die zweite Schnittstelle am distalen Ende übermittelt und der Haltearm Daten, die dieses Gewicht repräsentieren, speichert und entsprechend verarbeitet. Zusätzlich oder alternativ ist vorzugsweise vorgesehen, dass bei Überschreitung bestimmter Schwellwerte ein Signalton ausgegeben wird. Zudem ist bevorzugt, dass an den Gelenken Leuchtmittel vorgesehen sind, die aufleuchten, wenn ein vorbestimmter Schwellwert eines Gelenks überschritten wird. Wird also ein Gelenk mit einem zu hohen Drehmoment belastet, wird ein Leuchtmittel an diesem Gelenk ausgelöst und ein Operateur kann auf diese Weise wahrnehmen, welches Gelenk einer Entlastung bedarf, um eine stabile Pose zu erreichen.

Zum Übertragen dieser Daten weisen die erste und/oder zweite Schnittstelle vorzugsweise Übertragungsmittel auf zum Übertragen der von dem Sensor oder den Sensoren erfassten Daten. Diese Übertragungsmittel umfassen vorzugsweise die eingangs genannten Schnittstellen, wie insbesondere Bluetooth^{®}, USB, RS-232 oder ähnliche.

Gemäß einer weiteren bevorzugten Ausgestaltung weist der Haltearm eine Erkennungseinheit zum Erkennen eines mit der zweiten Schnittstelle gekoppelten Assistenzsystems auf, wobei die Bedieneinrichtung dazu angepasst ist, die Gelenke in Abhängigkeit des mit der zweiten Schnittstelle gekoppelten Assistenzsystems freizugeben oder zu arretieren. Eine derartige Erkennungseinheit weist vorzugsweise einen Barcode-Scanner, einen QR-Code-Scanner oder einen RFID-Scanner auf. Vorzugsweise ist ein an der zweiten Schnittstelle gekoppeltes Assistenzsystem mit einem entsprechenden Barcode, QR-Code oder einem RFID-Chip ausgestattet, welcher eine Identifikation des Assistenzsystems sowie vorzugsweise Informationen über dieses enthält. Hierdurch ist der Haltearm in der Lage, zu erkennen, welches Assistenzsystem an der zweiten Schnittstelle gekoppelt ist und so ein Freigeben bestimmter Gelenke teilweise oder vollständig zu verhindern. Beispielsweise ist an der zweiten Schnittstelle ein Endoskop angeordnet. Der RFID-Chip des Endoskops enthält Informationen über dieses Endoskop, wie insbesondere die geometrischen Abmaße des Endoskops. Diese Informationen werden von der Erkennungseinheit erkannt und an die Bedieneinrichtung des Haltearms und/oder die externe Steuereinheit weitergegeben. Die Bedieneinrichtung ist dazu ausgebildet, solche Posen des Haltearms zu verhindern, in welchen das Endoskop mit dem Haltearm kollidieren würde. Ferner kann die Bedieneinrichtung zusätzlich dazu ausgebildet sein, solche Positionen des Haltearms zu verhindern, indem das Endoskop mit beispielsweise einem Operationstisch oder weiteren Gegenständen kollidiert.

Die Erkennungseinheit ist vorzugsweise ferner dazu ausgebildet, ein Instrument, welches in den Operationsbereich eingeführt wird, zu erkennen und Daten, die dieses Instrument repräsentieren, an der ersten Schnittstelle bereitzustellen. Die Erkennungseinheit ist vorzugsweise ferner dazu ausgebildet, ein Instrument, welches aus dem Operationsbereich entfernt wird, zu erkennen, und Daten, die dieses Instrument repräsentieren, an der ersten Schnittstelle bereitzustellen. Das Instrument weist dazu bevorzugt einen Sender, etwa einen RFID-Chip auf, der mittels eines entsprechenden Empfängers, etwa RFID-Sensors, der in der Erkennungseinheit vorgesehen ist, kommuniziert. Dadurch ist es möglich festzustellen, zu welchem Zeitpunkt welches Instrument in den Operationsbereich eingeführt wird, und ob dieses den Operationsbereich wieder verlassen hat. Wird beispielsweise am Ende eines Operationsvorgangs festgestellt, dass sieben Instrumente zum Operationsbereich hingeführt wurden, jedoch nur sechs aus diesem entfernt wurden, kann dies ein Indiz dafür sein, dass sich noch ein Instrument im Operationsbereich befindet, wodurch ein Risiko für den Patienten hervorgerufen werden kann.

Gemäß einer weiteren bevorzugten Ausführungsform weist der Haltearm eine Kamera auf, die vorzugsweise am distalen Ende angeordnet ist, wobei die Kamera dazu vorgesehen ist, ein Operationsfeld zu beobachten und mit der ersten Schnittstelle zum Übertragen von Bilddaten an der ersten Schnittstelle gekoppelt ist. Dadurch ist auf vorteilhafte Art und Weise das Operationsfeld beobachtbar. Die Kamera ist durch die Kopplung an dem Haltearm besonders nah am Operationsfeld angeordnet und hat "freie Sicht" auf das Operationsfeld. Zudem ist ihre Position mittels des Haltearms verstellbar. Die Kamera kann auch dazu eingesetzt werden, Gegenstände wie etwa weitere chirurgische Instrumente und dergleichen in der Nähe des Haltearms zu beobachten und so eine Kollision mit diesen zu verhindern. Nimmt die Kamera beispielsweise wahr, dass der Haltearm zu nah an ein im Operationsfeld befindliches Instrument gebracht wird, kann vorgesehen sein, dass die Bedieneinrichtung ein oder mehrere Gelenke arretiert, sodass eine Kollision verhindert wird. In einem solchen Fall ist es bevorzugt, dass die Bedieneinrichtung einen Controller aufweist, der mit einer dafür eingerichteten Bilderkennungssoftware ausgestattet ist. Die Kamera kann in den Haltearm selbst integriert und fest mit diesem verbunden sein, oder die Kamera ist Teil eines Exoskops, welches mit der zweiten Schnittstelle gekoppelt ist. Die Kamera ist vorzugsweise als Full-HD Kamera oder als 3D-Kamera ausgebildet. Ist die Kamera Teil eines Exoskops, ist diese an der zweiten Schnittstelle an eine Spannungsschnittstelle, eine Lichtleiterschnittstelle und eine Datenschnittstelle angeschlossen. In der Verwendung wird die Kamera eines Exoskops typischerweise etwa 25 cm bis 75 cm von einem Operationsfeld entfernt angeordnet. Indem die Kamera des Exoskops direkt an der distalen Schnittstelle gekoppelt ist, können Daten an der proximalen Schnittstelle übertragen werden, beispielsweise an ein OP-System. Nun kann ein Anwender Bilder, welche mittels der Kamera aufgenommen werden, über einen entsprechenden 2D- oder 3D-Monitor des OP-Systems betrachten. Da es möglich ist, mittels des Haltearms die Kamera beziehungsweise die Linse der Kamera relativ nah am Operationsfeld zu positionieren, sind bei Verwendung entsprechender Monitore große Vergrößerungen von beispielsweise 12facher Vergrößerung einfach zu realisieren. Dadurch ist es nicht mehr erforderlich, ein großes und umständliches Operationsmikroskop einzusetzen, allein der Haltearm, welcher mit einer Kamera oder ein Exoskop ausgestattet ist, reicht aus, um das Operationsfeld zu beobachten. Indem der Haltearm zusätzlich Sensoren zur Bestimmung der Pose des Haltearms aufweist, ist bei Verwendung des erfindungsgemäßen Haltearms auch die Position bekannt, aus der die Bilder mittels der Kamera beziehungsweise der Kamera des Exoskops aufgenommen werden. Den aufgenommenen Bilddaten können so zeitliche Lageinformation zugeordnet werden. Somit ist es möglich, nach Abschluss der Operation Blickwinkel und Blickposition jedem einzelnen Bild zuzuordnen. Hieraus können postoperativ Schlussfolgerungen für die Dokumentation gezogen werden, aus welcher Perspektive welche operative Strategie gewählt wurde.

Vorzugsweise weist der Haltearm ferner ein Mikrofon auf, welches mit dem Controller und/oder Bedieneinrichtung gekoppelt ist, und der Controller und/oder die Bedieneinrichtung weist eine entsprechende Spracherkennungssoftware auf, sodass durch das Mikrofon empfangene Audiosignale in Stell- und Steuersignale für den Haltearm umgesetzt werden. So ist es bevorzugt, bei Empfang eines entsprechenden Audiosignals über das Mikrofon eine an dem Haltearm angeordnete oder an diesem integriert vorgesehene Kamera so zu steuern, dass diese ein Standbild, einen sogenannten Snapshot, aufnimmt und an der zweiten Schnittstelle an den Haltearm übergibt, sodass Daten, die diesen Snapshot repräsentieren, an der ersten Schnittstelle des Haltearmes in ein System übertragen werden können. Dadurch ist es möglich, dass der Operateur während der Operation einen Befehl gibt, etwa das Wort "Snapshot" ausspricht und durch eine Spracherkennungssoftware, welche in dem Controller vorgesehen ist, ein Signal von dem Controller an die Kamera gesendet wird, einen Snapshot aufzunehmen und Daten, die diesen repräsentieren, an der zweiten Schnittstelle bereitzustellen. Zu diesen Snapshots können durch den Haltearm ferner Positionsdaten übergeben werden.

Ist ferner in einem Operationssaal, in dem der Haltearm eingesetzt wird, ein Navigationssystem vorhanden, kann der Haltearm über die erste Schnittstelle mit diesem Navigationssystem verbunden werden. Solche Navigationssysteme sind beispielsweise von der Fa. Karl Storz GmbH & Co. KG, Tuttlingen, Deutschland oder auch der Fa. Olympus Deutschland GmbH, Hamburg, Deutschland erhältlich. In einem solchen Fall wird vorzugsweise an der Basis des Haltearms ein optischer Lokalisator befestigt, der mit dem Navigationssystem zusammenwirkt. Die Navigationskamera des Navigationssystems erfasst so den Haltearm und auch das Operationsfeld. Auch der Patient ist mit Lokalisatoren ausgestattet, sodass eine Patientenlage im Raum durch das Navigationssystem erfassbar ist. Allerdings sind die definierten Arbeitsräume eines Navigationssystems in der Regel begrenzt. Wird nun eine Kamera oder ein Exoskop an dem Haltearm vorgesehen, ist es möglich, die Kamera bzw. das Exoskop auch außerhalb des Arbeitsraums des Navigationssystems zu positionieren und die Lage der Kamera bzw. des Exoskops mittels des Haltearms zu bestimmen. Dadurch kann der begrenzte Arbeitsraum des Navigationssystems von zusätzlichen Werkzeugen freigehalten werden und das Navigationssystem verbessert genutzt werden. Ferner können Positionsdaten der Kamera zu den Daten, die aufgenommene Bilder repräsentieren, abgespeichert und mit Daten des Navigationssystems verknüpft werden. In einer weiteren bevorzugten Ausgestaltung ist an der zweiten Schnittstelle ein Sicherungselement vorgesehen, welches mit der Bedieneinrichtung gekoppelt ist, derart, dass die Bedieneinrichtung alle Gelenke arretiert, wenn das Sicherungselement eine fehlerhafte Kopplung zwischen dem Assistenzsystem und der zweiten Schnittstelle anzeigt. Ein derartiges Sicherungselement kann als elektronisches Sicherungselement oder als mechanisches Sicherungselement ausgebildet sein. Beispielsweise kann vorgesehen sein, dass bei korrekter Kopplung zwischen dem Assistenzsystem und der zweiten Schnittstelle ein Stromkreis geschlossen wird. In einer Alternative ist an dem Assistenzsystem ein Magnet angeordnet, und die zweite Schnittstelle weist einen entsprechenden Sensor auf, der dazu eingerichtet ist, das Magnetfeld des an dem Assistenzsystem angeordneten Sensors zu detektieren. Weitere Alternativen sind hier denkbar. Auch hierdurch wird die Sicherheit des Haltearms weiter verbessert. Ist ein Assistenzsystem nicht korrekt mit der zweiten Schnittstelle gekoppelt, werden alle Gelenke arretiert, und der Haltearm kann nicht bewegt werden. Die Gefahr, dass ein Haltearm mit einem nicht korrekt gekoppelten Assistenzsystem während einer Operation verwendet wird, kann dadurch verringert werden.

In einem dritten Aspekt der Offenbarung wird die eingangs genannte Aufgabe durch ein Verfahren zum Positionieren eines an einen Haltearm, vorzugsweise einen Haltearm nach einer der vorstehend beschriebenen bevorzugten Ausführungsformen eines Haltearms gemäß dem ersten oder zweiten Aspekt, gekoppelten chirurgischen mechatronischen Assistenzsystems und/oder chirurgischen Instruments, wobei der Haltearm wenigstens sechs Freiheitsgrade aufweist, mit den Schritten gelöst: Halten einer Pose des Haltearms, Erfassen eines Kontakts eines Bedieners mit einem ersten Armsegment des Haltearms; Freigeben eines dem ersten Armsegment zugeordneten ersten Gelenks, solange der Kontakt erfasst wird; Arretieren des ersten Gelenks, sobald der Kontakt nicht mehr erfasst wird. Durch ein solches Verfahren wird ein Verfahren zum Positionieren eines an einem Haltearm gekoppelten Assistenzsystems und/oder chirurgischen Instruments bereitgestellt, mittels dessen dieses intuitiv und auf einfache Art und Weise durch einen Bediener positioniert werden kann.

In einem Ruhezustand wird vorzugsweise die Pose des Haltearms gehalten. Erst bei Erfassen eines Kontakts zwischen einem Bediener und einem ersten Armsegment wird das zugeordnete erste Gelenk solange der Kontakt erfasst wird freigegeben. Bei Abbruch des Kontakts wird das Gelenk arretiert. Dadurch ist das Gelenk solange freigegeben, wie Kontakt zwischen dem Bediener und dem Armsegment besteht, sodass der Bediener bei Kontakt mit dem Armsegment dieses und sich daran anschließende Armsegmente bewegen kann, um das Assistenzsystem und/oder das chirurgische Instrument im Raum zu positionieren. Diese Art der Positionierung ist intuitiv, wodurch auch die Sicherheit eines chirurgischen Eingriffs unter Verwendung eines derartigen Haltearms verbessert ist, da Fehlbedienung vermieden werden kann. Vorzugsweise wird das Verfahren in der Kopfchirurgie ausgeführt. Vorzugsweise umfasst das Verfahren den Schritt Halten eines Endoskops während einer kopfchirurgischen Behandlung. Insbesondere im Gebiet der Kopfchirurgie ist eine genaue Positionierung erforderlich, da ein Raum zum Manipulieren von Gewebe begrenzt ist und je nach Position eine Manipulation von Gewebe hochsensibel ist.

Vorzugsweise weist das Verfahren ferner die Schritte auf: Erfassen eines Kontakts eines Bedieners mit einem zweiten Armsegment des Haltearms; Freigeben eines dem zweiten Armsegment zugeordneten zweiten Gelenks, solange der Kontakt erfasst wird; und Arretieren des zweiten Gelenks, sobald der Kontakt nicht mehr erfasst wird. Die Schritte werden bevorzugt gleichzeitig oder nach den oben genannten Schritten ausgeführt. Es ist möglich und bevorzugt, dass ein Bediener nur ein Armsegment oder zwei oder mehr Armsegmente kontaktiert. Daher werden alle den jeweils kontaktierten Armsegmenten zugeordneten Gelenke freigegeben. Hierdurch ist eine besonders einfache und rasche Positionierung des Assistenzsystems und/oder chirurgischen Instruments möglich.

In einer bevorzugten Weiterbildung wird der Kontakt an zwei im Wesentlichen gegenüberliegenden Seiten des Armsegments erfasst. Hierdurch ist es möglich, einen unbeabsichtigten Kontakt von einem beabsichtigten Greifen zu unterscheiden und so unbeabsichtigte Kontakte, bei denen der Bediener ein Armsegment beispielsweise nur mit einem Arm oder einem Handrücken berührt, ohne den Kontakt absichtlich herbeiführen zu wollen, auszuschließen. Allein wenn der Kontakt auf zwei gegenüberliegenden Seiten erfasst wird, was in der Regel aufgrund eines Ergreifens des entsprechenden Armsegments geschieht, wird das zugeordnete Gelenk freigegeben.

Gemäß einer weiteren bevorzugten Ausgestaltung des Verfahrens wird eine Intensität des Kontakts erfasst und abhängig von der Intensität das Gelenk teilweise oder vollständig freigegeben. Vorzugsweise wird das Gelenk in Abhängigkeit der Intensität des Kontakts stufenlos teilweise bis vollständig freigegeben. Ergreift beispielsweise ein Bediener das entsprechende Armsegment nur leicht und bringt eine nur geringe Kraft auf, wird das Gelenk nur teilweise freigegeben, derart, dass das Gelenk gegen einen Widerstand beweglich ist. Wird hingegen fest zugegriffen und demnach eine hohe Kraft aufgebracht, wird das Gelenk vollständig freigegeben, sodass das Armsegment im Wesentlichen widerstandsfrei beweglich ist. Hierdurch ist weiterhin eine intuitive Bedienung möglich, und ein Bediener kann durch die Kraft, die er beim Greifen aufbringt, die Freigabe der Gelenke steuern. Beispielsweise kann durch ein kurzes Antippen das Gelenk nur kurz freigegeben werden, wodurch eine Feinpositionierung des Arms erreichbar ist.

Ferner umfasst das Verfahren vorzugsweise den Schritt Ausgeben eines Signals zum Anzeigen des Kontakts zwischen dem Bediener und dem Armsegment. Durch diesen Schritt erhält der Bediener eine direkte Rückmeldung über den Kontakt und dadurch über die Freigabe des zugeordneten Gelenks.

Es soll verstanden werden, dass der Haltearm gemäß dem ersten Aspekt der Offenbarung und das Verfahren gemäß dem dritten Aspekt der Offenbarung gleiche und ähnliche Aspekte haben. Daher wird für die bevorzugten Ausführungsformen des Verfahrens sowie deren Vorteile auch auf die obige Beschreibung zum Haltearm vollumfänglich Bezug genommen.

In einem vierten Aspekt der Offenbarung wird die eingangs genannte Aufgabe durch ein Verfahren zum Steuern eines an einem Haltearm gekoppelten mechatronischen Assistenzsystems, insbesondere unter Verwendung eines Haltearms gemäß einer der vorstehend beschriebenen bevorzugten Ausführungsformen eines Haltearms, zur Navigation während einer chirurgischen Behandlung, mit den Schritten gelöst: Koppeln eines mechatronischen Assistenzsystems mit einer zweiten Schnittstelle des Haltearms an seinem distalen Ende; Übertragen von elektrischer Energie und Signalen von einer ersten Schnittstelle des Haltearms an seinem proximalen Ende; wobei das Übertragen mittels einer Übertragungseinrichtung, welche innerhalb des Haltearms angeordnet ist und die erste Schnittstelle mit der zweiten Schnittstelle zum Übertragen von Energie und Signalen zwischen den Schnittstellen verbindet, ausgeführt wird.

Durch ein derartiges Verfahren zum Steuern eines an einem Haltearm gekoppelten mechatronischen Assistenzsystems sind insbesondere die Sicherheit eines verwendeten Haltearms und die Sicherheit von mit diesem ausgeführten chirurgischen Schritte verbessert.

Vorzugsweise umfasst das Verfahren ferner die Schritte: Erfassen von Stellungen von Gelenken des Haltearms; Bestimmen einer Pose des Haltearms unter Verwendung der erfassten Stellung der Gelenke; und Bereitstellen von Daten, die die bestimmte Pose repräsentieren, an der ersten Schnittstelle. Die an der ersten Schnittstelle bereitgestellten Daten werden vorzugsweise mittels dieser an eine externe Steuereinheit übergeben, in welcher die Daten weiter verarbeitet und/oder ausgewertet werden. Eine derartige externe Steuereinheit kann beispielsweise als üblicher PC ausgebildet sein, oder als OP-System.

Weiterhin ist bevorzugt, dass das Verfahren die Schritte aufweist: Erfassen von auf Gelenke des Haltearms wirkende Drehmomenten; Bestimmen einer an dem distalen Ende des Haltearms angreifenden Kraft; und Bereitstellen von Daten die die bestimmte Kraft repräsentieren, an der ersten Schnittstelle. Auch diese Daten werden vorzugsweise an eine externe Steuereinheit übergeben. Die externe Steuereinheit kann diese Daten weiter verarbeiten und/oder auswerten. Beispielsweise kann die externe Steuereinheit ein Signal ausgeben, wenn ein Grenzwert einer auf den Haltearm wirkenden Kraft überschritten wird.

In einer weiteren bevorzugten Ausgestaltung weist das Verfahren die Schritte auf: Erkennen eines mit der zweiten Schnittstelle gekoppelten Assistenzsystems; Freigeben und Arretieren von Gelenken des Haltearms in Abhängigkeit des erkannten Assistenzsystems; Bereitstellen von Daten, die das erkannte Assistenzsystem repräsentieren, an der ersten Schnittstelle. Durch diese Schritte sind die Sicherheit des Haltearms und die Verwendung dieses Haltearms in einem chirurgischen Verfahren weiter verbessert. Sobald eine kritische und/oder "unzulässige" Pose mit dem Haltearm erreicht wird beziehungsweise der Haltearm in eine derartige Pose bewegt wird, werden entsprechende Gelenke arretiert, sodass diese kritische beziehungsweise "unzulässige" Pose nicht erreicht werden kann. Dadurch ist die Sicherheit des Haltearms weiter verbessert.

Weiterhin weist das Verfahren bevorzugt die Schritte auf: Erkennen, mittels einer Erkennungseinheit, ein in einen Operationsbereich eingeführtes Instrument; Bereitstellen von Daten an der proximalen Schnittstelle, die das Instrument repräsentieren und indizieren, dass das Instrument in den Operationsbereich eingeführt wurde. Gemäß diesen Verfahrensschritten werden Instrumente, die nicht an dem distalen Ende des Haltearms gekoppelt werden, mittels der Erkennungseinheit erkannt. Die Erkennungseinheit weist dazu vorzugsweise einen Empfänger, etwa einen RFID-Sensor auf, während das oder die in den Operationsbereich gebrachten Instrumente einen Sender, etwa ein RFID-Tag, aufweisen. Es ist auch möglich, dass der Haltearm ein Near-Field-Sensor aufweist, und das Instrument ein Near-Field-Chip aufweist. Ebenso sind andere Sender-Empfänger-Modelle möglich. Durch dieses Verfahren wird folglich erkannt, wann ein Instrument in den Operationsbereich eingeführt wird. Dabei werden an der ersten Schnittstelle Daten bereitgestellt, die das Instrument repräsentieren und angeben, um welches Instrument es sich handelt, als auch Daten, die angeben, dass das Instrument in den Operationsbereich eingeführt wurde. Vorzugsweise werden diese Daten gespeichert.

In einer bevorzugten Weiterbildung ist ferner vorgesehen, dass das Verfahren die Schritte aufweist: Erkennen, mittels einer Erkennungseinheit, eines aus dem Operationsbereich ausgeführtes Instruments; Bereitstellen von Daten an der ersten Schnittstelle, die das Instrument repräsentieren und indizieren, dass das Instrument aus dem Operationsbereich ausgeführt wurde. Hier gilt die obige Beschreibung entsprechend. Allerdings wird hier gemäß diesen Verfahrensschritten erkannt, ob und wann das entsprechende Instrument aus dem Operationsbereich ausgeführt wurde. Ergibt sich nach Abschluss der Operation eine Diskrepanz zwischen eingeführten und ausgeführten Instrumenten, deutet dies darauf hin, dass sich Instrumente noch im Operationsbereich befinden. Hierdurch kann ein Risiko bei der Operation verringert werden.

In einer bevorzugten Ausgestaltung weist das Verfahren die Schritte: Erfassen von Bilddaten eines Operationsfelds; und Bereitstellen der Bilddaten an der ersten Schnittstelle auf. Die Bilddaten umfassen sowohl zweidimensionale als auch dreidimensionale Bilddaten und werden vorzugsweise mittels einer Kamera erfasst, die am distalen Ende des Haltearms angeordnet ist.

In einer bevorzugten Weiterbildung sind die Bilddaten mit Positionsdaten des Assistenzsystems verknüpft. Das Assistenzsystem weist vorzugsweise eine Kamera auf. Indem die Bilddaten mit Positionsdaten des Assistenzsystems verknüpft sind, insbesondere Metadaten des Bildes Daten über die Position der Kamera umfassen, ist es möglich zu bestimmen, aus welcher Position das entsprechende Bild aufgenommen wurde.

In einer bevorzugten Weiterbildung des Verfahrens weist dieses die Schritte auf: Erfassen eines Audiosignals mittels eines Mikrofons; Erkennen eines Sprachbefehls in dem Audiosignal; Wandeln des Sprachbefehls in ein Stellsignal für das Assistenzsystem; und Bereitstellen des Stellsignals an der zweiten Schnittstelle. Vorzugsweise wird zur Verarbeitung des Audiosignals eine bekannte Spracherkennungssoftware eingesetzt. Bestimmte Sprachbefehle können mit bestimmten Stellsignalen verknüpft sein. So ist es beispielsweise möglich, dass ein Sprachbefehl: "Screenshot" einem Stellsignal zugeordnet ist, welches eine an der zweiten Schnittstelle angeordnete Kamera veranlasst, ein Bild aufzunehmen. Weitere Stellsignale sind denkbar und bevorzugt.

Weiterhin ist bevorzugt, dass das Verfahren ferner die folgenden Schritte aufweist: Bestimmen, ob das Assistenzsystem korrekt mit der zweiten Schnittstelle gekoppelt ist; Arretieren aller Gelenke des Haltearms, wenn das Assistenzsystem nicht korrekt mit der zweiten Schnittstelle gekoppelt ist. Auch hierdurch ist die Sicherheit weiter verbessert. Zusätzlich kann vorgesehen sein, dass das Verfahren den Schritt aufweist: Ausgeben eines Alarmsignals, wenn das Assistenzsystem nicht korrekt mit der zweiten Schnittstelle gekoppelt ist. Ein solches Alarmsignal kann beispielsweise als Audiosignal oder visuelles Signal ausgebildet sein.

In einer bevorzugten Ausgestaltung des Verfahrens weist dieses ferner den Schritt auf: Anzeigen einer Repräsentation von an der ersten und/oder zweiten Schnittstelle übergebenen Daten. Eine derartige Repräsentation kann beispielsweise die Anzeige einer Identifikation eines Assistenzsystems, welches an dem distalen Ende angeordnet ist, umfassen. Es können Informationen über dieses Assistenzsystem angezeigt werden, wie beispielsweise Fähigkeiten, Restriktionen, Stellparameter und dergleichen. Ferner ist es auch möglich, eine Pose des Haltearms darzustellen oder auf einzelne Gelenke wirkende Kräfte.

In einer weiteren bevorzugten Ausgestaltung weist das Verfahren die Schritte auf: Speichern der an der ersten Schnittstelle bereitgestellten Daten; Erstellen eines Operationsprotokolls unter Verwendung der gespeicherten Daten. Vorzugsweise werden sämtliche erfassten und bereitgestellten Daten gespeichert und auf Basis dieser Daten das Operationsprotokoll erstellt. Die erfassten Daten umfassen Positions- und Lagedaten der Gelenke, sowie auf das distale Ende wirkende Kräfte. Auf Basis dieser Daten ist es möglich, jede einzelne Pose des Haltearms während einer Operation nachzuvollziehen und so die Lage eines an dem Haltearm angeordneten Assistenzsystems im Raum während des gesamten Operationsprozesses zu kennen. Hieraus ist nachvollziehbar, wie das Assistenzsystem relativ zum Patienten bewegt wurde und dadurch, welche Schritte vorgenommen worden sind. Gemäß diesem Aspekt der Offenbarung ist es also nicht erforderlich, dass ein Operateur jeden einzelnen Schritt mit protokolliert, dies kann im Nachhinein durch Auslesen der Daten und Verarbeiten dieser Daten erfolgen. Hierdurch ist weiterhin die Sicherheit verbessert, da die Gefahr von Protokollfehlern verringert wird.

Ferner umfasst das Verfahren vorzugsweise die Schritte: Speichern aller an der ersten Schnittstelle bereitgestellten Daten; Erzeugen einer DICOM-Datei auf Basis der gespeicherten Daten. Eine DICOM-Datei ist ein bekanntes Datenaustauschformat für OP-Systeme und kann durch eine Vielzahl von Systemen verwendet werden, um eine Operation im Nachhinein zu analysieren und nachzubereiten. Eine DICOM-Datei kann zudem verwendet werden, um in einer elektronischen Patientenakte abgelegt zu werden. Vorzugsweise werden diese Schritte automatisch nach Abschluss eines Operationsvorgangs ausgeführt.

Es soll verstanden werden, dass der Haltearm gemäß dem zweiten Aspekt der Offenbarung sowie das Verfahren gemäß dem vierten Aspekt der Offenbarung gleiche und ähnliche Unteraspekte haben. Insofern wird für einzelne Ausgestaltungen des Haltearms sowie für die Vorteile des Verfahrens auf die obige Beschreibung zu dem ersten Aspekt der Offenbarung vollumfänglich verwiesen.

Nachstehend wird die Erfindung anhand eines Ausführungsbeispiels unter Bezugnahme auf die beiliegenden Figuren näher erläutert. Dabei zeigen:
- Figur 1: eine Seitenansicht eines Haltearms, bei dem die Kontaktmittel zu sehen sind;
- Figur 2: eine teilweise aufgebrochene Ansicht des Haltearms aus Figur 1;
- Figur 3: eine schematische Darstellung des vierten Armsegments;
- Figur 4: eine weitere schematische Darstellung des vierten Armsegments;
- Figur 5: den Haltearm aus Figur 1, mit einer externen Steuereinheit gekoppelt;
- Figur 6: eine Draufsicht auf die Schnittstelle am proximalen Ende des Haltearms;
- Figur 7: eine perspektivische Ansicht eines externen Energiespeichers;
- Figur 8: eine perspektivische schematische Darstellung des ersten Armsegments mit einer mechanischen Schnittstelle zur Kopplung des Haltearms an eine Normschiene eines Operationstisches;
- Figur 9: eine perspektivische Darstellung des siebten Armsegments samt Schnittstelle am distalen Ende;
- Figur 10: eine Draufsicht auf die Schnittstelle am distalen Ende des Haltearms;
- Figur 11a: ein Ausführungsbeispiel des Haltearms mit einer teilweise strukturierten Oberfläche;
- Figur 11b: ein Ausführungsbeispiel eines Haltearms mit einer teilweise farblich gestalteten Oberfläche;
- Figur 12: einen Teilschnitt durch eine Bremse in einem Gelenk des Haltearms;
- Figur 13: ein Flussdiagramm eines Verfahrens gemäß einem ersten Ausführungsbeispiel; und
- Figur 14: ein Flussdiagramm eines Verfahrens gemäß einem zweiten Ausführungsbeispiel.

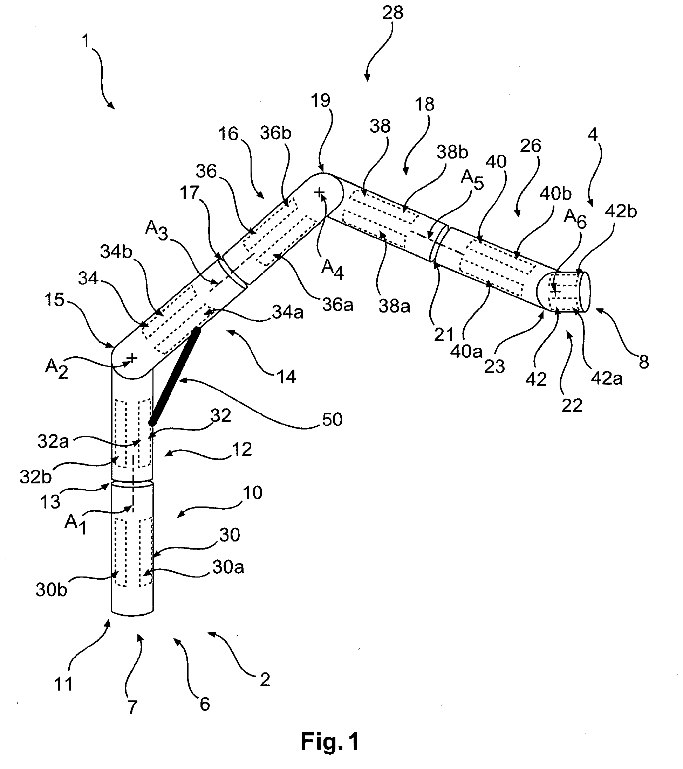

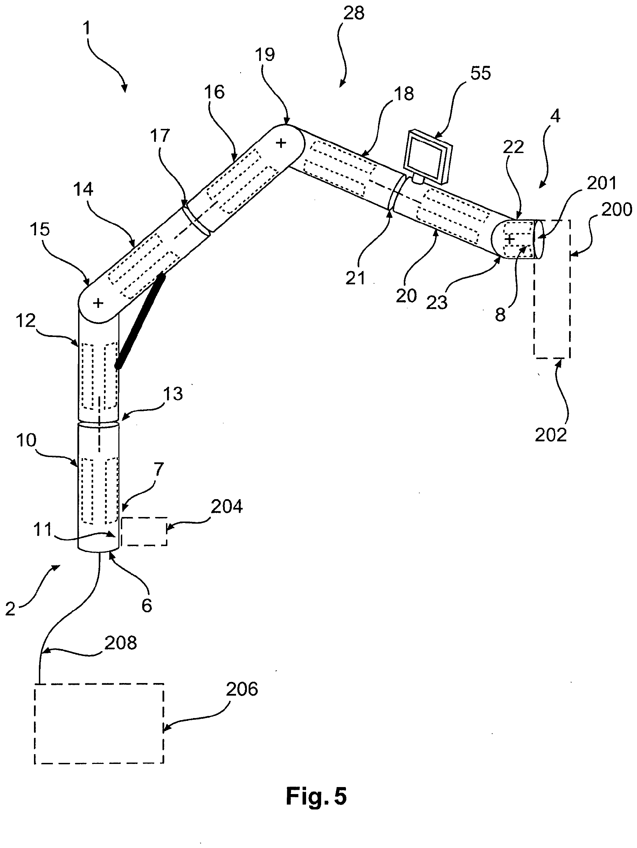

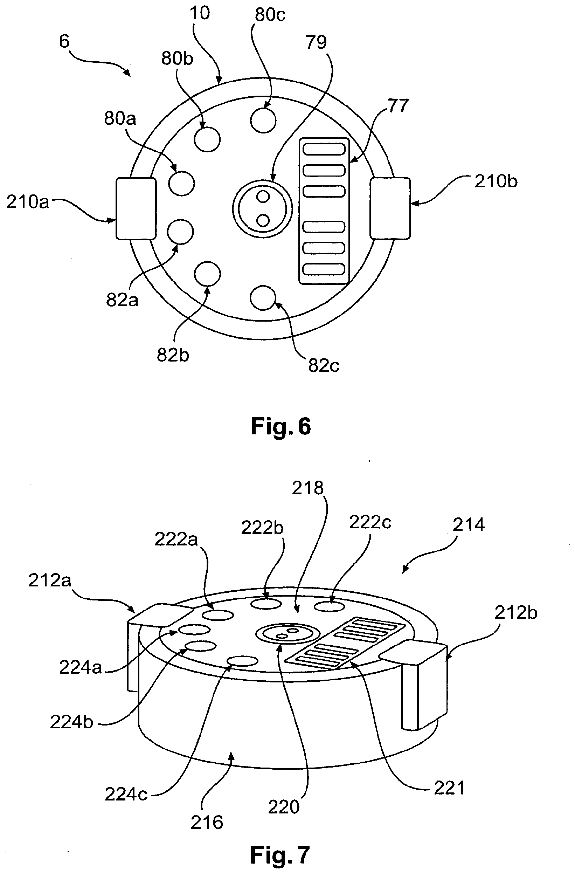

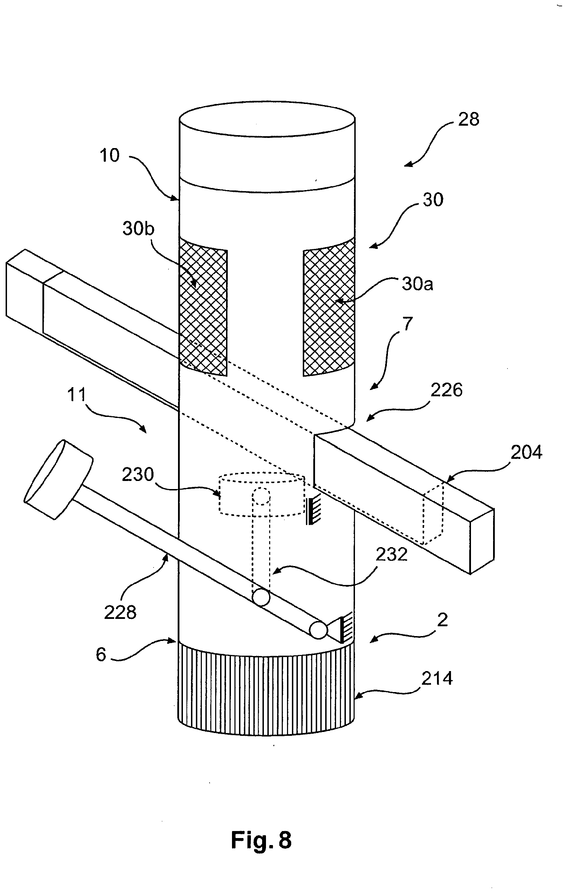

Figur 1 zeigt einen Haltearm 1 für medizinische Zwecke, insbesondere zum Halten eines chirurgischen mechatronischen Assistenzsystems und/oder chirurgischen Instruments. Der Haltearm 1 weist ein proximales Ende 2 und ein distales Ende 4 auf. An dem proximalen Ende 2 sind eine erste Schnittstelle 6 und eine mechanische Schnittstelle 7 ausgebildet, welche mit Bezug auf die Figuren 6 und 8 genauer beschrieben werden. Die Schnittstelle 7 dient dazu, den Haltearm 1 an einer Basis, wie insbesondere an einem Operationstisch, zu befestigen. Die Schnittstelle 7 dient zur Übergabe von Energie sowie zur Kopplung des Haltearms 1 mit einer externen Steuereinheit (vgl. Fig. 5). An dem distalen Ende 4 ist eine zweite Schnittstelle 8 vorgesehen, über die ein mechatronisches Assistenzsystem und/oder ein chirurgisches Instrument, wie insbesondere ein Manipulator, an den Haltearm 1 koppelbar ist. Vorzugsweise wird hier ein Manipulator zum Halten und Manipulieren eines Endoskops angeordnet.

Der Haltearm 1 gemäß Figur 1 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen. Die sieben Armsegmente 10, 12, 14, 16, 18, 20, 22 sind jeweils mit Gelenken 11, 13, 15, 17, 19, 21, 23 miteinander gekoppelt, wobei das nullte Gelenk 11 den Haltearm 1 mit der Basis (in Fig. 1 nicht gezeigt, s. Fig. 7) koppelt. Die Gelenke 13, 15, 17, 19, 21, 23 sind gemäß diesem Ausführungsbeispiel sämtlich als rotatorische Gelenke mit jeweils einem Freiheitsgrad ausgebildet. Gemäß diesem Ausführungsbeispiel ist dem nullten Segment 10 das nullte Gelenk 11 zugeordnet, dem ersten Armsegment 12 das erste Gelenk 13 zugeordnet, dem zweiten Armsegment 14 das zweite Gelenk 15 zugeordnet, dem dritten Armsegment 16 das dritte Gelenk 17 zugeordnet, dem vierten Armgelenk 18 das vierte Gelenk 19 zugeordnet, dem fünften Armsegment 20 das fünfte Gelenk 21 zugeordnet, und dem sechsten Armsegment 22 ist das sechste Gelenk 23 zugeordnet. Das Gelenk 11 ist als translatorisches Gelenk ausgebildet, sodass das Armsegment 10 teleskopartig verlängerbar ist, um so den Haltearm 1 in der Höhe zu verstellen, wie später mit Bezug auf Figur 8 erläutert werden wird. Die Gelenke 13, 15, 17, 19, 21, 23 weisen jeweils Schwenkachsen A₁, A₂, A₃, A₄, A₅, A₆ auf, wobei jeweils benachbarte Gelenke Schwenkachsen haben, die senkrecht zueinander sind. Hierdurch wird eine einfache Positionierung des distalen Endes 4 im Raum erreicht.

Der Haltearm 1 gemäß Figur 1 weist ferner eine Bedieneinrichtung 28 auf. Mittels der Bedieneinrichtung 28 ist der Haltearm 1 in eine gewünschte Pose verbringbar, wobei die Bedieneinrichtung 28 dazu eingerichtet ist, bei Kontakt zwischen einer Bedienperson und einem der sieben Armsegmente das zugeordnete Gelenk freizugeben. Dazu weist die Bedieneinrichtung 28 gemäß diesem Ausführungsbeispiel sieben Kontaktabschnitte 30, 32, 34, 36, 38, 40, 42 auf, wobei an jedem Armsegment 10, 12, 14, 16, 18, 20, 22 jeweils ein Kontaktmittel 30, 32, 34, 36, 38, 40, 42 angeordnet ist. So ist am nullten Armsegment 10 ein nulltes Kontaktmittel 30 angeordnet, am ersten Armsegment 12 ein erstes Kontaktmittel 32, am zweiten Armsegment 14 ein zweites Kontaktmittel 34, am dritten Armsegment 16 ein drittes Kontaktmittel 36, am vierten Armsegment 18 ein viertes Kontaktmittel 38, am fünften Armsegment 20 ein fünftes Kontaktmittel 40 und am sechsten Armsegment 22 ein sechstes Kontaktmittel 42 angeordnet.

Ferner ist gemäß diesem Ausführungsbeispiel vorgesehen, dass jedes Kontaktmittel 30, 32, 34, 36, 38, 40, 42 jeweils zwei im Wesentlichen gegenüberliegend angeordnete Kontaktmittelelemente 30a, 30b, 32a, 32b, 34a, 34b, 36a, 36b, 38a, 38b, 40a, 40b, 42a, 42b aufweist. Die Kontaktmittel 30, 32, 34, 36, 38, 40, 42 dienen dazu, einen Kontakt eines Bedieners mit dem entsprechenden Armsegment 10, 12, 14, 16, 18, 20, 22 zu erfassen. Beim Ergreifen eines Armsegments 10, 12, 14, 16, 18, 20, 22 kommt der Bediener in Kontakt mit beiden Kontaktmittelelementen 30a, 30b bis 42a, 42b, und nur bei Kontakt mit beiden Kontaktmittelelementen 30a, 30b bis 42a, 42b eines Kontaktmittels 30 bis 42 wird das zugeordnete Gelenk freigegeben. Das heißt, beim Ergreifen des ersten Armsegments 12 und gleichzeitigem Inkontaktkommen mit den beiden Kontaktmittelelementen 32a, 32b wird durch die Bedieneinrichtung 28 das erste Gelenk 13 freigegeben. Dadurch ist es möglich, dass der Bediener den Haltearm 1 beziehungsweise die Armsegmente 12 bis 22 um die Achse A₁ verschwenken kann. Bei Loslassen eines der beiden oder beider Kontaktmittelelemente 32a, 32b wird das Gelenk 13 wieder arretiert, und ein Verschwenken um die Achse A₁ ist nicht mehr möglich. Auch bei einer unbeabsichtigten Berührung nur eines der beiden Kontaktmittelelemente 32a, 32b, beispielsweise mit einem Arm oder Ellenbogen des Bedieners, wird das Gelenk 13 nicht freigegeben, und der Haltearm 1 bleibt im arretierten Zustand und hält seine Pose.

Entsprechendes gilt für das zweite Armsegment 14. Auch hier weist das zweite Kontaktmittel 34 zwei Kontaktmittelelemente 34a, 34b auf, die im Wesentlichen gegenüberliegend umfänglich an dem Armsegment 14 vorgesehen sind. Bei Ergreifen dieses Armsegments 14 und Inkontaktkommen mit den beiden Kontaktmittelelementen 34a, 34b wird dieser Kontakt durch die Bedieneinrichtung 28 erfasst, und das dem Armsegment 14 zugeordnete Gelenk 15 freigegeben. Nun ist ein Verschwenken um die Achse A₂ möglich, sodass das distale Ende 4, bezogen auf Figur 1, nach oben beziehungsweise unten verschwenkt werden kann. Gleichzeitig bleiben alle weiteren Gelenke 13, 17, 19, 21, 23 arretiert, sodass in diesen keine Bewegung stattfindet.

Die Bedieneinrichtung 28 kann dazu einen Controller oder Mikroprozessor aufweisen, der dazu eingerichtet ist, einen Kontakt zwischen Kontaktmittelelementen 30a, 30b bis 42a, 42b zu erfassen und in elektrische Signale zu übertragen.

Die Kontaktmittel 30 beziehungsweise die Kontaktmittelemente 30a, 30b bis 42a, 42b sind gemäß diesem Ausführungsbeispiel als berührungsempfindliche Sensoren ausgebildet und erfassen einen Druck eines Kontakts zwischen dem Bediener und dem entsprechenden Kontaktmittelelement 30a, 30b bis 42a, 42b. Vorzugsweise sind die Kontaktmittelelemente 30a, 30b bis 42a, 42b als kapazitive berührungsempfindliche Sensoren ausgebildet.

Bei dem dargestellten Haltearm 1 ist es auch möglich, dass ein Bediener zwei Armsegmente, beispielsweise das Armsegment 14 und das Armsegment 18, gleichzeitig ergreift und so gleichzeitig die Kontaktmittelelemente 34a, 34b und 38a, 38b kontaktiert. Folglich werden die Gelenke 15 und 19 freigegeben, und ein Verschwenken sowohl um die Achse A2 als auch um die Achse A4 ist möglich. Bei dieser gleichzeitigen Freigabe ist es möglich, eine Winkelorientierung der Armsegmente 18 und 20 im Raum beizubehalten, während nur die Armsegmente 34, 36 verschwenkt werden. So ist auch eine translatorische Bewegung des distalen Endes 4 möglich. In einer bevorzugten Ausgestaltung des Haltearms werden bei dem gleichzeitigen Kontaktieren von zwei Armsegmenten, gemäß diesem Beispiel der Armsegmente 14 und 18 nicht die Gelenke 15 und 19 freigegeben, sondern alle zwischen diesen Armsegmenten 14 und 18 liegenden Gelenke, also gemäß diesem Ausführungsbeispiel die Gelenke 17 und 19. Das Gelenk 15 bleibt arretiert. Der Haltearm 1 kann nun so in seiner Pose verändert werden, dass eine Rotation um die Achse A3 und die Achse A4 möglich ist. Dies ist eine besonders intuitive Bedienung des Haltearms. Entsprechend werden, beispielsweise bei dem Kontakt zwischen Bediener und den Haltearmsegmenten 12 und 20, die Gelenke 15, 17, 19 und 21 freigegeben.

Weiterhin ist in der Figur 1 zu erkennen, dass der Haltearm 1 über eine Gewichtskompensationseinrichtung 50 verfügt. Die Gewichtskompensationseinrichtung 50 weist gemäß diesem Ausführungsbeispiel ein Gasdruckfederelement auf, welches mit dem Armsegment 14 und dem Armsegment 12 gekoppelt ist. Alternativ kann die Gewichtskompensationseinrichtung auch einen Seilzug und/oder ein ausbalanciertes Gegengewicht aufweisen. Bei dem Haltearm 1 gemäß Figur 1 lastet auf dem Gelenk 15 um seine Rotationsachse A2 das größte Moment. Folglich ist es bevorzugt, genau dieses Gelenk 15 mittels der Gewichtskompensationseinrichtung 50 abzustützen. Bei Freigabe des Gelenks 15, durch Kontaktieren des Armsegments 14, wird also ein Gewicht, welches auf dem Armsegment 14 lastet, aufgrund der weiteren Armsegmente 16, 18, 20, 22 und eines an der Schnittstelle 8 angeordneten Manipulators durch die Gewichtskompensationseinrichtung 50 abgestützt, sodass das distale Ende 4 bei Ergreifen des Segments 14 nicht sofort "absackt".

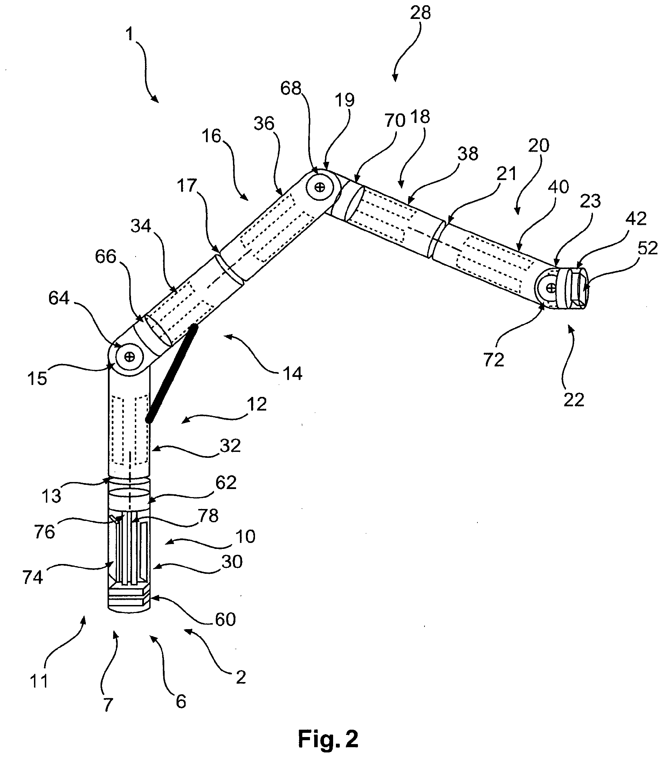

Der Haltearm 1 (vgl. Fig. 2) weist eine Erkennungseinheit 52 zum Erkennen eines mit der zweiten Schnittstelle 8 gekoppelten Assistenzsystems auf, wobei die Bedieneinrichtung 28 dazu angepasst ist, die Gelenke 11, 13, 15, 17, 19, 21, 23 in Abhängigkeit des mit der zweiten Schnittstelle 8 gekoppelten Assistenzsystems freizugeben oder zu arretieren. Eine derartige Erkennungseinheit 52 weist vorzugsweise einen Barcode-Scanner, einen QR-Code-Scanner oder einen RFID-Scanner auf. Vorzugsweise ist ein an der zweiten Schnittstelle 8 gekoppeltes Assistenzsystem mit einem entsprechenden Barcode, QR-Code oder einem RFID-Chip ausgestattet, welcher eine Identifikation des Assistenzsystems sowie vorzugsweise Informationen über dieses enthält. Hierdurch ist der Haltearm 1 in der Lage, zu erkennen, welches Assistenzsystem an der zweiten Schnittstelle 8 gekoppelt ist und so ein Freigeben bestimmter Gelenke 11, 13, 15, 17, 19, 21, 23 teilweise oder vollständig zu verhindern. Beispielsweise ist an der zweiten Schnittstelle 8 ein Endoskop angeordnet. Der RFID-Chip des Endoskops enthält Informationen über dieses Endoskop, wie insbesondere die geometrischen Abmaße des Endoskops. Diese Informationen werden von der Erkennungseinheit erkannt und an die Bedieneinrichtung 28 des Haltearms 1 und/oder die externe Steuereinheit weitergegeben. Die Bedieneinrichtung 28 ist dazu ausgebildet, solche Posen des Haltearms 1 zu verhindern, in welchen das Endoskop mit dem Haltearm kollidieren würde. Ferner kann die Bedieneinrichtung 28 zusätzlich dazu ausgebildet sein, solche Posen des Haltearms 1 zu verhindern, in denen das Endoskop mit beispielsweise einem Operationstisch oder weiteren Gegenständen kollidieren würde.

In Figur 2 sind bei dem Haltearm 1 zusätzlich zu den bereits in Figur 1 gezeigten Elementen die Bremsen 60, 62, 64, 66, 68, 70, 72 dargestellt, mittels derer die Gelenke 11, 13, 15, 17, 19, 21, 23 freigebbar und arretierbar sind. Gleiche und ähnliche Elemente sind mit gleichen Bezugszeichen wie in Figur 1 versehen, und insofern wird vollumfänglich auf die obige Beschreibung Bezug genommen. Auch wenn in Figur 2 die Bezugszeichen an den Kontaktmittelelementen der Kontaktmittel 30, 32, 34, 36, 38, 40, 42 aus Klarheitsgründen nicht gezeigt sind, sind diese gleichwohl vorhanden, wie sich aus einem Vergleich der Figuren 1 und 2 ergibt.

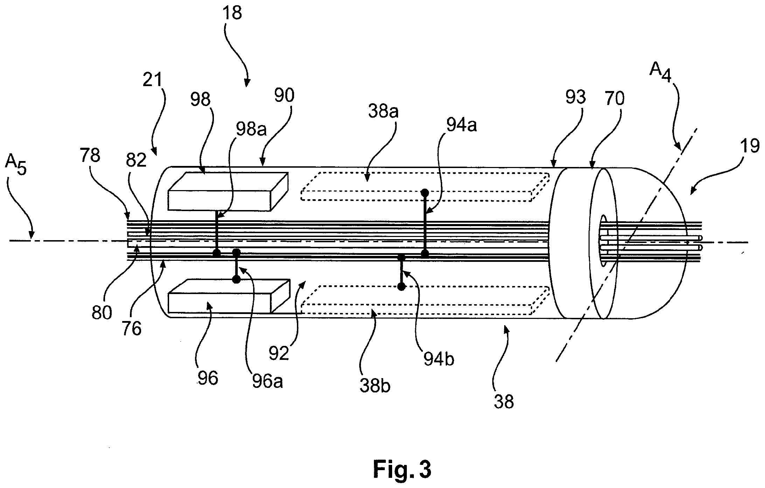

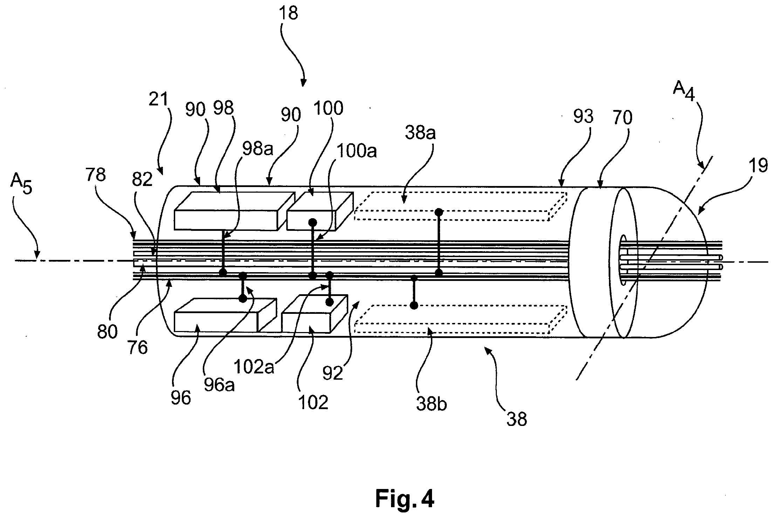

Jedem Gelenk 11, 13, 15, 17, 19, 21, 23 ist jeweils eine Bremse 60, 62, 64, 66, 68, 70, 72 zugeordnet. Dem Gelenk 11 ist die Bremse 60 zugeordnet, dem Gelenk 13 die Bremse 62, dem Gelenk 15 die Bremse 64, dem Gelenk 17 die Bremse 66, dem Gelenk 19 die Bremse 68, dem Gelenk 21 die Bremse 70 und dem Gelenk 23 die Bremse 72. Alle Bremsen 60 bis 72 sind als elektromagnetische Bremsen mit einem Permanentmagnet ausgebildet, derart, dass diese unbestromt in einen arretierten Zustand vorgespannt sind. Der Permanentmagnet ist derart ausgelegt, dass dieser das jeweilige Gelenk alleine arretieren kann und die Pose des Haltearms 1 gehalten wird. In dem nullten Armsegment 10 ist eine elektronische Steuereinheit 74 vorgesehen. Diese ist über ein Bussystem 76 (in Figur 2 nur im Armsegment 10 gezeigt; vgl. Fig. 3 und 4) mit allen Kontaktmitteln 30 bis 42 der Bedieneinrichtung 28 sowie mit allen Bremsen 60 bis 72 gekoppelt. Zur Energieversorgung der Bremsen 60 bis 72 sowie der Kontaktmittel 30 bis 42 ist ferner eine Energieleitung 78 vorgesehen, die über die Schnittstelle 6 am proximalen Ende 2 des Haltearms 1 mit einer Energiequelle koppelbar ist.

Die Figuren 3 und 4 zeigen zwei verschiedene Ausführungsformen eines Armsegments, wobei in den Figuren 3 und 4 jeweils exemplarisch das vierte Armsegment 18 dargestellt ist. Es soll erkannt werden, dass auch die anderen Armsegmente 10, 12, 14, 16, 20, 22 ebenso ausgebildet sein können.

Das Armsegment 18 weist einen Armsegmentkörper 90 auf (in den Fig. 1 und 2 nicht gezeigt; es soll verstanden werden, dass jedes Armsegment 10 bis 22 einen Armsegmentkörper hat), der gemäß den Figuren 3 und 4 im Wesentlichen stabförmig beziehungsweise zylindrisch ausgebildet ist. Im Inneren weist der Armsegmentkörper 90 einen Hohlraum 92 auf, in dem verschiedene Elemente, wie etwa die Bremse 70, angeordnet sind. Schematisch dargestellt in den Figuren 3 und 4 sind die Gelenke 19, 21 sowie die beiden Schwenkachsen A₄, A₅ der Gelenke 19, 21, die jeweils mit dem Haltearmsegment 18 zusammenwirken. Dem Haltearmsegment 18 ist das Gelenk 19 zugeordnet (vgl. obige Beschreibung zu den Fig. 1 und 2). Der Armsegmentkörper 90 weist eine äußere Oberfläche 93 auf, die im Wesentlichen zylindrisch ausgebildet ist. Der Armsegmentkörper 90 ist beispielsweise aus einem Metall wie insbesondere Aluminium oder Titan, einer Legierungen auf Basis von Aluminium oder Titan, oder einem Faserverbundwerkstoff, wie etwas GFK oder CFK, gebildet und vorzugsweise in Leichtbauweise ausgelegt.

Das Armsegment 18 weist gemäß den Figuren 3 und 4 das Kontaktmittel 38 auf, welches Teil der Bedieneinrichtung 28 ist (vgl. Fig. 1 und 2). Das Kontaktmittel 38 weist zwei Kontaktmittelelemente 38a, 38b auf, die als berührungsempfindliche Sensoren ausgebildet sind und flächenbündig in der äußeren Oberfläche 93 des Armsegments 18 angeordnet sind. Die beiden Kontaktmittelelemente 38a, 38b sind im Wesentlichen gegenüberliegend bezogen auf die Achse A₅ angeordnet, sodass ein Bediener beim Ergreifen des Armsegments 18 in Kontakt mit beiden Kontaktmittelelementen 38a, 38b kommt, wie dies oben beschrieben ist.

Die beiden Kontaktmittelelemente 38a, 38b sind mittels Leitungen 94a, 94b mit dem Bussystem 76 gekoppelt. Über das Bussystem 76 sind die Kontaktmittelelemente 38a, 38b mit der elektronischen Steuereinheit 74 (vgl. Fig. 2) gekoppelt und über diese wiederum mit der Bremse 70, sodass die Bremse 70 durch die Bedieneinrichtung 28 freigegeben wird, wenn ein Bediener mit den Kontaktmittelelementen 38a, 38b in Kontakt kommt.

Neben dem Bussystem 76 sind innerhalb des Armsegmentkörpers 90 ein Energieübertragungssystem 78 sowie ein Kabelkanal 80 und ein Arbeitskanal 82 angeordnet. Mittels des Energieübertragungssystems 78 sind die Kontaktmittelelemente 38a, 38b sowie die Bremse 70 an eine Energieversorgung angeschlossen.

Alternativ oder zusätzlich ist in jedem Armsegment ein Elektronikmodul 96 angeordnet, welches über eine Leitung 96a mit dem Bussystem 76 gekoppelt ist. In einem solchen Fall wirken die Kontaktmittelelemente 38a, 38b, welche über die Leitung 94a, 94b mit dem Datenbussystem 76 verbunden sind, nur mit dem Elektronikmodul 96 zusammen, welches den durch die Kontaktmittelelemente 38a, 38b erfassten Kontakt in ein Stellsignal für die Bremse 70 umwandelt und dieses Stellsignal über das Bussystem 76 an die Bremse 70 sendet zum Freigeben des Gelenks 19. Ist in jedem Armsegment ein derartiges Elektronikmodul 96 angeordnet, ist ein im Wesentlichen modularer Aufbau des Haltearms 1 realisiert, und die einzelnen Armsegmente 10 bis 22 sind unabhängig von der elektronischen Steuereinheit 74, welche im proximalen Armsegment 10 angeordnet ist.

Der Kabelkanal 80 dient dazu, Kabel, die vom proximalen Ende 2 bis zum distalen Ende 4 verlaufen, um insbesondere die Schnittstelle 8 zu versorgen, führen zu können. Der Arbeitskanal 82 dient dazu, Schläuche oder Lichtleiter und dergleichen aufzunehmen, die je nach Art des Manipulator, der an der Schnittstelle 8 angeordnet ist, benötigt werden. Ist beispielsweise an der Schnittstelle 8 ein Endoskop angeordnet, wird vorzugsweise durch den Arbeitskanal 82 ein Lichtleiter geführt, der ein Bild, welches eine Endoskopkamera aufnimmt, übertragen kann. Der Arbeitskanal 82 dient demnach dazu, je nach Anwendungsgebiet entsprechende Übertragungsmittel aufzunehmen.

Weiterhin ist in dem Armsegment 18 ein Sensor 98 angeordnet. Vorzugsweise ist in jedem Armsegment 10 bis 22 ein Sensor angeordnet, und es soll verstanden werden, dass die Sensoren in den Armsegmenten 10, 12, 14, 16, 20 und 22 ebenso ausgebildet sein können wie der Sensor 98 im Armsegment 18. Der Sensor 98 ist vorzugsweise als Beschleunigungssensor ausgebildet. Indem in jedem Armsegment ein solcher Beschleunigungssensor vorgesehen ist, ist es möglich, zu jeder Zeit die Pose des Haltearms 1 zu bestimmen. Dazu ist der Sensor 98 über eine Leitung 98a mit dem Datenbussystem 76 gekoppelt, sodass die von dem Sensor 98 erfassten Daten an die elektronische Steuereinheit 74 übertragen werden, welche dann aus allen Sensordaten aus allen Armsegmenten die Pose des Haltearms 1 bestimmt. Ferner ist durch das Vorsehen eines derartigen Sensors 98 auch die absolute und relative Position eines an der Schnittstelle 8 angeordneten Endeffektors beziehungsweise Manipulators bestimmbar. Auch ist es möglich, wenn der Haltearm 1 an einem Operationstisch angeordnet ist, eine Bewegung dieses Operationstischs zu erkennen. Erfassen alle Sensoren in allen Armsegmenten eine Bewegung in dieselbe Richtung, ist dies ein Indikator dafür, dass der gesamte Haltearm 1 unter Beibehaltung seiner Pose bewegt wurde, beispielsweise dadurch, dass der Operationstisch oder eine Operationstischplatte relativ zu einer Säule der Operationstisches rotiert oder verschoben wurde. Auch eine solche Bewegung ist mittels der Sensoren 98 erfassbar. Ferner können äußere Impulse, wie etwa Stöße auf den Haltearm 1, erfasst werden.

Gemäß Figur 4 ist zusätzlich in dem Armsegment 18 gemäß Figur 3 ein Speicherelement 100 vorgesehen sowie eine Energieerzeugungseinrichtung 102. Das Speicherelement dient dazu, elektrische Energie zu speichern, sodass Sensoren, die in dem spezifischen Armsegment vorgesehen sind, auch in einem vom Stromnetz abgekoppelten Zustand mit einem Strom versorgt werden können. Dies ist insbesondere dann erforderlich, wenn beispielsweise Bump-Sensoren einer Kamera oder dergleichen vorgesehen sind, die auch in einem Ruhezustand des Haltearms funktionieren müssen, um einen möglichen Schaden am Haltearm 1 zu detektieren. Die Energieerzeugungseinrichtung 102 dient dazu, eine Energie für beispielsweise einen Laser, ein Ultraschallgerät oder dergleichen, welche am Haltearm gekoppelt sind, bereitzustellen. Zusätzlich oder alternativ kann die Energieerzeugungseinrichtung 102 auch eine Einrichtung zum Versorgen des Speicherelements 100 aufweisen, beispielsweise ein Energy harvesting element, welches Bewegungsenergie oder Energie aufgrund eines Magnetfelds, beispielsweise induktiv, in elektrische Spannung für das Speicherelement 100 wandelt.

Figur 5 illustriert erneut den Haltearm 1, welcher bereits mit Bezug auf die Figuren 1 und 2 beschrieben worden ist. In Figur 5 ist der Haltearm 1 in ein System eingebunden dargestellt. An dem distalen Ende 4 ist mittels der Schnittstelle 8 ein chirurgisches mechatronisches Assistenzsystem 200 angeordnet, welches mit der Schnittstelle 8 über eine Schnittstelle 201 gekoppelt ist. Sowohl das chirurgische mechatronische Assistenzsystem 200 als auch die Schnittstelle 201 sind in Figur 5 nur schematisch dargestellt. Es soll verstanden werden, dass das chirurgische mechatronische Assistenzsystem 200 beispielsweise als Endoskop oder Laparoskop oder dergleichen ausgebildet sein kann. Das Assistenzsystem 200 weist einen Arbeitsabschnitt 202 auf, der beispielsweise die Spitze des Endoskops sein kann. An dem proximalen Ende 2 ist der Haltearm 1 gemäß Figur 5 über die mechanische Schnittstelle 7 mit einer Basis 204 gekoppelt. Die Basis 204 ist hier ebenfalls nur schematisch dargestellt. Sie kann beispielsweise als eine Normschiene eines Operationstisches ausgebildet sein.

Die erste Schnittstelle 6 ist gemäß diesem Ausführungsbeispiel mit einer externen Steuereinheit 206 gekoppelt. Dazu ist die Schnittstelle 6 mittels eines Kabels 208 mit der externen Steuereinheit 206 verbunden. Die externe Steuereinheit 206 ist gemäß diesem Ausführungsbeispiel als OP-System ausgebildet, welches beispielsweise einen herkömmlichen Computer aufweist sowie eine Eingabe-Ausgabe-Schnittstelle zur Bedienung des OP-Systems. Das OP-System weist vorzugsweise Software-Komponenten auf, die dazu ausgebildet sind, Daten, die von dem Haltearm 1 an der Schnittstellt 6 übergeben werden, zu speichern und zu verarbeiten.

Je nach Ausgestaltung der Schnittstelle 6 kann auch vorgesehen sein, dass diese drahtlos mit dem OP-System 206 kommuniziert, beispielsweise über Bluetooth^{®}, Wi-Fi^{®} oder Ähnliches.

Gemäß diesem Ausführungsbeispiel weist der Haltearm 1 ferner ein Anzeigemittel 55 auf, welches gemäß diesem Ausführungsbeispiel als LCD Display ausgebildet ist. Das Anzeigemittel 55 ist mit einer Steuereinheit verbunden und zeigt Repräsentationen von Daten an, die an der ersten Schnittstelle 6 oder zweiten Schnittstelle 8 übergeben werden. Beispielsweise werden auf dem Anzeigemittel ein Gewicht eines Assistenzsystems 200, welches an der Schnittstelle 8 gekoppelt ist, angezeigt. Alternativ wird auf dem Anzeigemittel eine Repräsentation der Pose des Haltearms dargestellt, mit entsprechenden Belastungen an einzelnen Gelenken. Weitere Möglichkeiten sind hier denkbar. Es ist auch denkbar, dass hier Warnhinweise angezeigt werden.

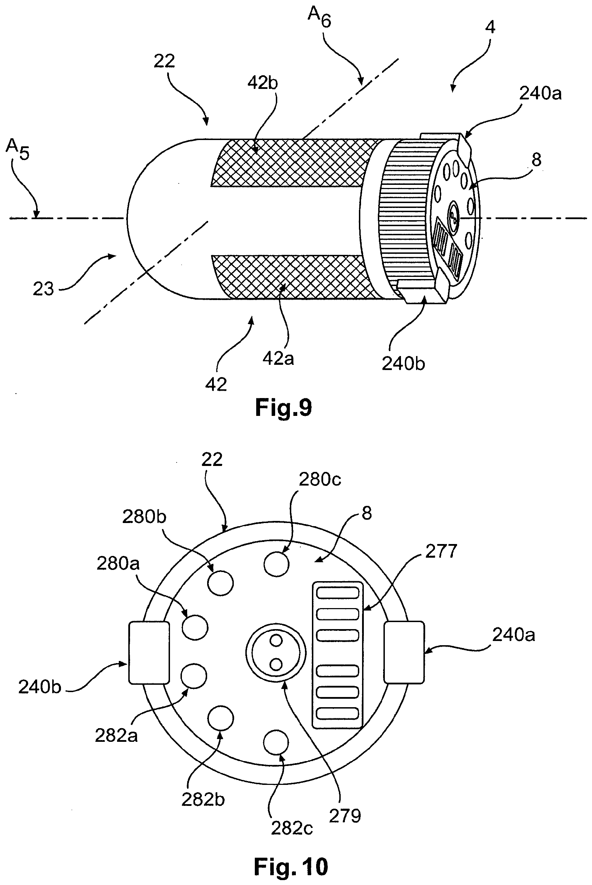

Die Schnittstelle 6 (s. Fig. 6) weist einen Anschluss 77 für das Bussystem 76 auf. Über diesen Anschluss 77 sind Daten, welche von Sensoren (vgl. Fig. 3 u. 4) sowie den Kontaktmittelelementen (vgl. Fig. 2 - 4) an das Bussystem 76 gegeben werden, an die externe Steuereinheit 206 übertragbar. Der Anschluss 77 kann dazu als USB-Schnittstelle, RS-232-Schnittstelle, Bluetooth^{®}-Schnittstelle, Wi-Fi^{®}-Schnittstelle oder dergleichen ausgebildet sein. Mittig an der Schnittstelle 6 ist ferner ein Anschluss 79 zum Übertragen von elektrischer Energie vorgesehen. Mittels dieses Anschlusses 79 ist der Haltearm 1 mit einer Energiequelle, beispielsweise dem Stromnetz, koppelbar. Weiterhin sind an der Schnittstelle 6 drei Auslässe 80a, 80b, 80c des Kabelkanals 80 (vgl. obige Beschreibung zu den Fig. 2, 3, u. 4) vorgesehen. Über diese Auslässe 80a, 80b, 80c sind Kabel, welche in den Kabelkanal 80 geführt sind, zugänglich. Zudem sind in der Schnittstelle 6 drei Auslässe 82a, 82b, 82c des Arbeitskanals 82 vorgesehen. Über die Auslässe 82a, 82b, 82c ist der Arbeitskanal 82 zugänglich. So ist beispielsweise mittels der Schnittstelle 6 ein Schlauch durch den Auslass 82b in den Arbeitskanal 82 führbar und durch diesen bis zur distalen Schnittstelle 8 (vgl. Fig. 10) führbar.

An einem umfänglichen Bereich des Armsegments 10 sind im Bereich der Schnittstelle 6 mechanische Kopplungsmittel 210a, 210b vorgesehen. Die Kopplungsmittel 210a, 210b korrespondieren mit Kopplungsmitteln 212a, 212b eines externen Energiespeichers 214 (s. Fig. 7). Der externe Energiespeicher 214 weist ein Gehäuse 216 auf, welches so gebildet ist, dass es sich proximal an das Armsegment 10 anfügen lässt. Der externe Energiespeicher 214 weist im Inneren Zellen zur Speicherung elektrischer Energie auf (in Fig. 7 nicht gezeigt). Der externe Energiespeicher 214 weist eine Schnittstelle 218 auf, die mit der Schnittstelle 6 des Haltearms 1 korrespondiert. Die Schnittstelle 218 weist einen Anschluss 220 auf, mittels dessen die in dem externen Energiespeicher 214 gespeicherte elektrische Energie über den Anschluss 79 der Schnittstelle 6 an den Haltearm 1 übertragbar ist. Ferner weist die Schnittstelle 218 einen Anschluss 221 auf, der mit der Schnittstelle 77 korrespondiert, zum Durchleiten von Signalen des Bussystems 76. Weiterhin weist der externe Energiespeicher Durchlässe 222a, b, c, 224a, b, c, auf, die mit den Auslässen 80a, b, c und 82a, b, c der Schnittstelle 6 korrespondieren, sodass durch den Kabelkanal 80 geführte Kabel auch durch den Energiespeicher 214 führbar sind sowie die Auslässe 82a, 82b, 82c des Arbeitskanals 80 ebenso an dem Energiespeicher 214 zugänglich sind.

Fig. 8 illustriert das Armsegment 10, welches das proximale Ende 2 des Haltearms bildet, sowie insbesondere die mechanische Schnittstelle 7. An der ersten Schnittstelle 6 ist der externe Energiespeicher 214 (vgl. Fig. 7) angeordnet, sodass der Haltearm 1 gemäß diesem Ausführungsbeispiel (Fig. 8) autark betrieben werden kann, ohne die Notwendigkeit, diesen mit einer externen Energiequelle zu verbinden. Eine Verbindung zu einer Steuereinheit 206 kann dennoch vorgesehen sein und ist bevorzugt. Das Armsegment 10 weist ein Kontaktmittel 30, welches zwei Kontaktmittelabschnitte 30a, 30b hat, auf (vgl. auch Fig. 2). Die Schnittstelle 7 ist gemäß diesem Ausführungsbeispiel als eine Ausnehmung 226 gebildet, die an der äußeren Kontur der Basis 204, die hier als Normschiene eines Operationstisches ausgebildet ist, entspricht. Die Basis 204 ist in die Ausnehmung 226 führbar, und an dem Armsegment 10 sind mechanische Klemmmittel 228 vorgesehen, zum Klemmen des Armsegments 10 gegen die Basis 204. Die Klemmmittel weisen einen linear geführten Klemmkörper 230 auf, der über ein Gestänge 232 mit einem Hebel 224 antreibbar ist. Das Gelenk 11, welches über das Kontaktmittel 30 der Bedieneinrichtung 28 freigebbar ist, ist folglich als translatorisches Gelenk 11 ausgerichtet. Die Bedieneinrichtung ist dazu über ein elektrisches Stellmittel, welches in Figur 8 nicht gezeigt ist, mit dem Hebel 228 gekoppelt, sodass das Klemmmittel 230 außer Eingriff von der Basis 204 bringbar ist.

Die Figuren 9 und 10 schließlich illustrieren die zweite Schnittstelle 8 am distalen Ende 2 des Haltearms 1. Während Figur 9 die Schnittstelle 8 in einer perspektivischen Ansicht samt dem Armsegment 22 zeigt, zeigt Figur 10 die Schnittstelle 8 in einer Frontalansicht.

Die Schnittstelle 8 ist im Wesentlichen korrespondierend zur Schnittstelle 6 ausgebildet. An seitlichen Abschnitten von dieser, an dem Armsegment 22, sind zwei Sicherungselemente 240a, 240b angeordnet. Mittels der Sicherungselemente ist bestimmbar, ob ein an der Schnittstelle 8 gekoppeltes Assistenzsystem 200 (s. Fig. 5) korrekt mit der Schnittstelle 8 gekoppelt ist. Die Sicherungselemente 240a, 240b dienen gleichzeitig als mechanische Kopplungsmittel zum mechanischen Koppeln mit dem Assistenzsystem 200, beispielsweise mittel Klemmung oder Verrastung.

An der Schnittstelle 8 ist ferner ein Anschluss 277 angeordnet, der mit dem Anschluss 77 der Schnittstelle 6 korrespondiert. Der Anschluss 277 ist mit dem Bussystem 76 gekoppelt, sodass Daten und Signale von dem Anschluss 77 über das Bussystem 76 zum Anschluss 77 übertragbar sind und umgekehrt. Ebenso ist ein Anschluss 279 an der Schnittstelle 8 vorgesehen, mittels dessen elektrische Energie von der Schnittstelle 8 auf das Assistenzsystem 200 übertragbar ist. Der Anschluss 279 korrespondiert mit dem Anschluss 79 der Schnittstelle 6, und die beiden Anschlüsse 279 und 79 sind mittels des Übertragungsmittels 78 gekoppelt, zum Übertragen von elektrischer Energie zwischen diesen beiden Anschlüssen 79, 279.

Ferner sind an der Schnittstelle 8 Auslässe 280a, 280b, 280c des Kabelkanals 80 vorgesehen, sodass durch diesen geführte Kabel an der Schnittstelle 8 zugänglich sind. Das Gleiche gilt für den Arbeitskanal 82, von dem an der Schnittstelle 8 drei Auslässe 282a, 282b, 282c vorgesehen sind. Durch diese Schnittstelle 8 ist ein Assistenzsystem 200 auf vorteilhafte Art und Weise mit dem Haltearm 1 koppelbar, ohne dass zusätzliche Übertragungsmittel oder Verkabelung an dem Haltearm 1 vorgesehen sein muss.

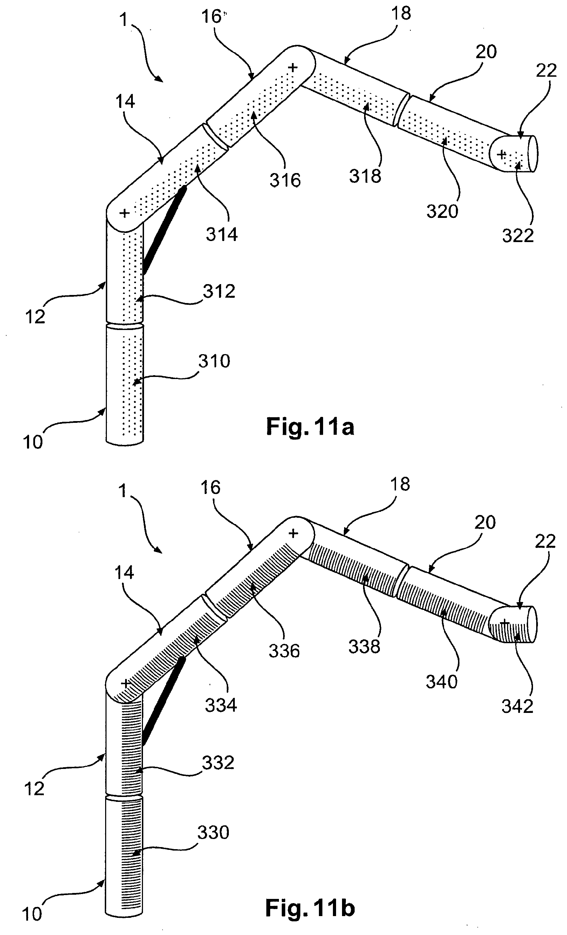

Die Figuren 11a und 11b zeigen zwei weitere Ausführungsbeispiele des Haltearms 1, der im Wesentlichen entsprechend dem ersten Ausführungsbeispiel gemäß den Figuren 1 und 2 ausgebildet ist. Zusätzlich zu den dort beschriebenen Merkmalen weist der Haltearm gemäß den Figuren 11a, 11b jeweils Ausrichtungsindikatoren 310, 312, 314, 316, 318, 320, 322, 330, 332, 334, 336, 338, 340, 342 auf. Gemäß Fig. 11a sind die Ausrichtungsindikatoren 310, 312, 314, 316, 318, 320, 322 als eine Oberflächenstruktur ausgebildet. Die einzelnen Armsegmente 10, 12, 14, 16, 18, 20, 22, welche im Wesentlichen eine zylindrische Grundform aufweisen, weisen etwa entlang einer halben Zylindermantelfläche eine Strukturierung auf. Die Strukturierung ist gemäß diesem Ausführungsbeispiel so angeordnet, dass die Ausrichtungsindikatoren 310, 312, 314, 316, 318, 320, 322 des Haltearms 1 in einer Grundpose des Haltearms 1, wie sie in Fig. 11a dargestellt ist, zum Operationsfeld hin ausgerichtet sind. Durch die Strukturierung, die als Ausrichtungsindikator 310, 312, 314, 316, 318, 320, 322, wirkt, kann ein Bediener taktil feststellen, ob ein Armsegment 10, 12, 14, 16, 18, 20, 22 in der Grundpose ausgerichtet ist, oder ob der Haltearm invertiert, das heißt, mit der strukturierten Oberfläche vom Operationsfeld weg ausgerichtet ist. Dies kann für eine Gewichtskompensation wichtig sein.

Alternativ dazu zeigt Fig. 11b ein Ausführungsbeispiel, bei dem die Ausrichtungsindikatoren 330, 332, 334, 336, 338, 340, 342 als farbliche Markierung ausgebildet sind. An den zum Operationsfeld hingewandten Seiten der Armsegmente 10, 12, 14, 16, 18, 20, 22 ist ein Farbverlauf vorgesehen, der durch einen Operateur visuell wahrnehmbar ist. Hierdurch kann der Operateur direkt erkennen, in welcher Orientierung sich der Haltearm 1 befindet.

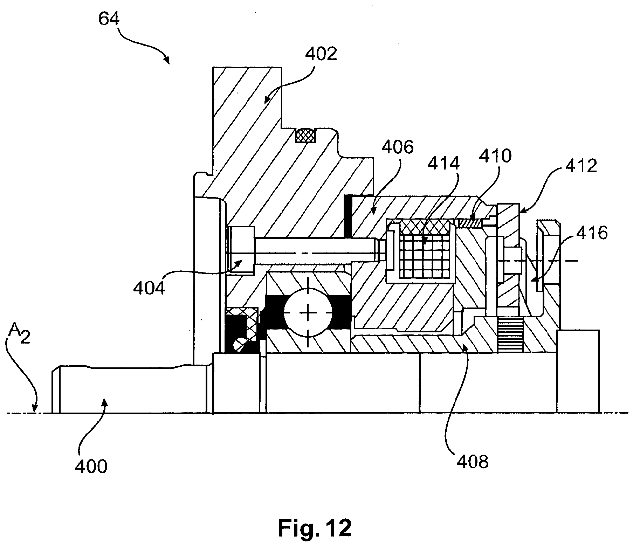

Fig. 12 zeigt schematisch eine Teilschnittdarstellung einer beispielhaften Bremse, wie sie beispielsweise als Bremse 64 in Gelenk 15 vorgesehen ist. Es soll verstanden werden, dass auch die weiteren Bremsen 60, 62, 66, 68, 70, 72 entsprechend ausgebildet sein können. Die Bremse 74 ist gemäß diesem Ausführungsbeispiel (Fig. 12) als elektromagnetische Permanentmagnetbremse ausgebildet. Ein erstes Gelenkelement 400, hier als Welle ausgebildet, ist starr mit dem Armsegment 12 verbunden, und ein zweites Gelenkelement 402 ist starr mit dem Armsegment 14 verbunden. Es soll verstanden werden, dass dies auch genau umgekehrt sein kann. Das zweite Gelenkelement 402 ist über eine Schraube 404 fest mit einem Gehäuse 406 der Bremse 64 gekoppelt. Eine Flanschnabe 408 ist entsprechend fest mit dem ersten Gelenk 400 gekoppelt. In dem Gehäuse 406 ist ein Permanentmagnet 410 angeordnet, der dazu dient einen Anker 412, der wiederum fest mit dem Flansch 408 verbunden ist, gegen das Gehäuse 406 zu pressen. Hierdurch wird eine Haftreibung hervorgerufen, die als Bremskraft wirkt. Im Gehäuse 406 ist ferner eine Erregerwicklung 414 angeordnet, die im bestromten Zustand ein dem Feld des Permanentmagneten 410 entgegenwirkendes Feld hervorruft. Durch ein Federelement 416, welches den Anker 412 in einem belüfteten Zustand vorspannt, wird dieser von dem Gehäuse 406 abgehoben und so wird die Bremse 64 belüftet. Eine Rotation des zweiten Gelenkelements 402 um das erste Gelenkelement 400 um die Achse A2 ist so möglich.

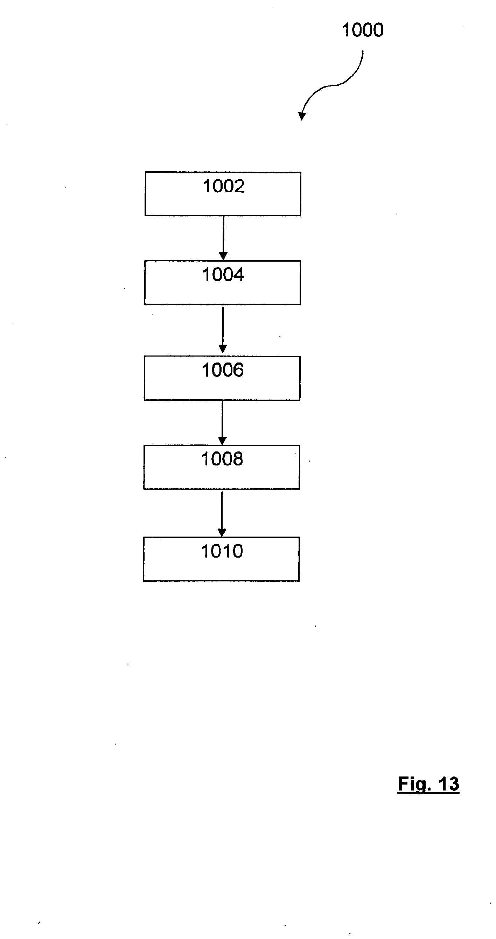

Figur 13 zeigt ein Verfahren 1000 zum Steuern eines an einem Haltearm 1 gekoppelten mechatronischen Assistenzsystems 200 gemäß am ersten Ausführungsbeispiel. Das Verfahren 1000 umfasst gemäß diesem Ausführungsbeispiel fünf Schritte, die nacheinander oder teilweise gleichzeitig ausgeführt werden. Beim ersten Schritt 1002 wird der Haltearm 1 an einer Normschiene eines Operationstischs befestigt und in Betrieb genommen. Dazu wird die erste Schnittstelle 4 mit einem OP-System 206 (siehe auch Figur 5) verbunden, sodass Daten und elektrische Energie an der ersten Schnittstelle 6 an den Haltearm 1 übertragen werden. In Schritt 1004 wird ein mechatronisches Assistenzsystem 200 mit einer zweiten Schnittstelle 8 des Haltearms 1 an seinem distalen Ende 4 gekoppelt. Die nachfolgenden Schritte 1006 bis 1010 werden dann vorzugsweise gleichzeitig ausgeführt. In Schritt 1006 werden elektrische Energie und Signale von der ersten Schnittstelle 6 des Haltearms 1 an einem proximalen Ende an den Haltearm übertragen. Von der ersten Schnittstelle 6 aus werden die Daten und elektrische Energie mittels einer Übertragungseinrichtung 76, 78 innerhalb des Haltearms 1 zu der zweiten Schnittstelle 8 übertragen. An der zweiten Schnittstelle am distalen Ende werden dann Daten und elektrische Energie bei Schritt 1010 an das Assistenzsystem übertragen, sodass das Assistenzsystem 200 betrieben werden kann.

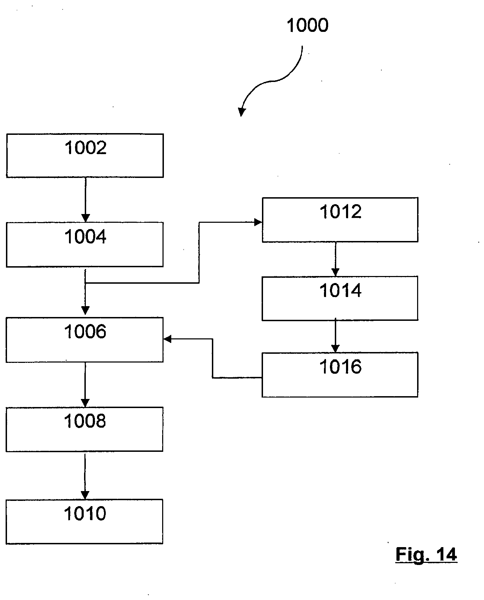

In Figur 14 ist ein weiteres Ausführungsbeispiel des Verfahrens 1000 dargestellt. Die Schritte 1002 bis 1010 sind entsprechend dem Ausführungsbeispiel gemäß Figur 13 ausgebildet und insofern wird vollumfänglich auf die obige Beschreibung Bezug genommen. Nach Schritt 1004 führt nun eine Abzweigung bei diesem Verfahren (siehe Figur 14) zu Schritt 1012. In Schritt 1012 werden Stellungen von Gelenken des Haltearms erfasst.

Aus den erfassten Stellungen der Gelenke wird in Schritt 1014 eine Pose des Haltearms unter Verwendung der erfassten Stellung der Gelenke bestimmt. Anschließend werden Daten, die die bestimmte Pose repräsentieren, bei Schritt 1016 bereitgestellt, und zwar an der ersten Schnittstelle 6 und werden in Schritt 1006 übertragen. Dadurch ist die Pose des Haltearms an der ersten Schnittstelle an ein OP-System übertragbar und kann dort verwendet werden.

Auf ähnliche Art und Weise werden die weiteren oben beschriebenen bevorzugten Ausführungsformen des Verfahrens ausgebildet, wobei die Schritte jeweils im Wesentlichen gleichzeitig und kontinuierlich ausgeführt werden können.

## Patentansprüche

1. Haltearm (1) für medizinische Zwecke, insbesondere zum Halten von chirurgischen mechatronischen Assistenzsystemen und/oder chirurgischen Instrumenten, mit
einem proximalen Ende (2) zum Befestigen des Haltearms (1) an einer Basis und einem distalen Ende (4) zum Aufnehmen eines chirurgischen mechatronischen Assistenzsystems und/oder chirurgischen Instruments;
wenigstens einem ersten und einem zweiten Armsegment (12, 14), wobei das erste Armsegment (12) mit einem ersten Gelenk (13) und das zweite Armsegment (14) mit einem zweiten Gelenk (15) verbunden ist, wobei jedes Gelenk (13, 15) freigebbar und arretierbar ist; und
einer Bedieneinrichtung (28) zum Verbringen des Haltearms (1) in eine gewünschte Pose, **dadurch gekennzeichnet, dass**
die Bedieneinrichtung (28) dazu eingerichtet ist bei Kontakt zwischen einer Bedienperson und einem der ersten und zweiten Armsegmente (12, 14) das zugeordnete Gelenk (13, 15) freizugeben, so dass bei Kontakt zwischen einer Bedienperson und dem ersten Armsegment (12) das erste Gelenk (13) freigegeben wird und bei Kontakt zwischen einer Bedienperson und dem zweiten Armsegment (14) das zweite Gelenk (15) freigegeben wird.

2. Haltearm nach Anspruch 1, wobei die Bedieneinrichtung (28) Kontaktmittel (30-42) aufweist, die dazu vorgesehen sind, dass ein Bediener mit diesen in Kontakt kommt, wobei ein erstes Kontaktmittel (32) der Bedieneinrichtung (28) an dem ersten Armsegment (12) angeordnet ist und ein zweites Kontaktmittel (34) an dem zweiten Armsegment (14) angeordnet ist.

3. Haltearm nach Anspruch 2, wobei jedes Kontaktmittel (30-42) zwei an dem Armsegment (10-22) im Wesentlichen gegenüberliegend angeordnete Kontaktmittelelemente (30a, 30b-42a, 42b) aufweist.

4. Haltearm nach Anspruch 2 oder 3, wobei die Kontaktmittelelemente (30a, 30b-42a, 42b) als Taster oder als berührungsempfindliche Sensoren ausgebildet sind.

5. Haltearm nach einem der vorstehenden Ansprüche, wobei die Bedieneinrichtung (28) dazu ausgebildet ist, in Abhängigkeit der Intensität des Kontakts das zugeordnete Gelenk (11-23) freizugeben.

6. Haltearm nach einem der vorstehenden Ansprüche, wobei die Bedieneinrichtung (28) bei Kontakt zwischen der Bedienperson mit sowohl dem ersten und als auch dem zweiten Armsegment (12, 14) alle zwischen diesen Armsegmenten angeordneten Gelenke (13, 15) freigibt.

7. Haltearm nach einem der vorstehenden Ansprüche, wobei die Gelenke (11-23) Bremsen aufweisen, mittels der die Gelenke (11-23) freigebbar und arretierbar sind, wobei die Bremsen vorzugsweise als elektromagnetische Bremsen ausgebildet sind und jeweils einen Permanentmagnet aufweisen, der die Bremse im unbestromten Zustand in den arretierten Zustand vorspannt.

8. Haltearm nach einem der vorstehenden Ansprüche, mit
einer ersten Schnittstelle (6) an dem proximalen Ende (2) zum Verbinden des Haltearm (1) mit einer Energiequelle sowie mit einer externen Steuereinheit (206) zum Übertragen von Signalen an den und von dem Haltearm (1);
einer zweiten Schnittstelle (8) an dem distalen Ende (4) zum Koppeln des Haltearms (1) mit dem Assistenzsystem (200) zum Steuern des Assistenzsystems (200); und
einer Übertragungseinrichtung (76, 78), welche innerhalb des Haltearms (1) angeordnet ist und die erste Schnittstelle (6) mit der zweiten Schnittstelle (8) zum Übertragen von Energie und Signalen zwischen den Schnittstellen (6, 8) verbindet.

9. Haltearm nach Anspruch 8, wobei die Übertragungseinrichtung ein Bussystem (76, 78) aufweist.

10. Haltearm nach einem der vorstehenden Ansprüche, wobei wenigstens ein Armsegment einen Sensor zum Erfassen einer Lage des Armsegments aufweist.

11. Haltearm nach Anspruch 8, mit einer Erkennungseinheit zum Erkennen eines mit der zweiten Schnittstelle gekoppelten Assistenzsystems, wobei die Bedieneinrichtung (28) dazu angepasst ist, die Gelenke (11, 13, 15, 17, 19, 21, 23) in Abhängigkeit des mit der zweiten Schnittstelle (8) gekoppelten Assistenzsystems (200) freizugeben oder zu arretieren.

12. Haltearm nach Anspruch 8, mit einer Kamera, die vorzugsweise am distalen Ende (4) angeordnet ist, wobei die Kamera dazu vorgesehen ist, ein Operationsfeld zu beobachten und mit der ersten Schnittelle (6) zum Übergeben von Bilddaten an der ersten Schnittstelle (6) gekoppelt ist.

13. Haltearm nach Anspruch 8, wobei an der zweiten Schnittstelle (8) ein Sicherungselement (240a, 240b) vorgesehen ist, welches mit der Bedieneinrichtung (28) gekoppelt ist, derart dass die Bedieneinrichtung (28) alle Gelenke (11, 13, 15, 17, 19, 21, 23) arretiert, wenn das Sicherungselement (240a, 240b) eine fehlerhafte Kopplung zwischen dem Assistenzsystem (200) und der zweiten Schnittstelle (8) anzeigt.

## Claims

1. A holding arm (1) for medical purposes, in particular for holding surgical mechatronic assistance systems (200) and/or surgical instruments, with
a proximal end (2) for attaching the holding arm (1) to a base and a distal end (4) for receiving a surgical mechatronic assistance system and/or surgical instrument;
at least one first and one second arm segment (12, 14), wherein the first arm segment (12) is connected to a first joint (13) and the second arm segment (14) is connected to a second joint (15), wherein each joint (13, 15) is releasable and lockable; and
an operating unit (28) for bringing the holding arm (1) into a desired pose, **characterized in that**
the operating unit (28) being configured to release upon contact between an operator and one of the first and second arm segments (14, 16) the associated joint (13, 15) such that upon contact between an operator and the first arm segment (12) the first joint (13) is released and upon contact between the operator and the second arm segment (14) the second joint (15) is released.

2. The holding arm according to claim 1, wherein the operating unit (28) comprises contact means (30-42), which are provided for an operator to come into contact with therewith, wherein a first contact means (32) of the operating unit (28) is arranged on the first arm segment (12) and a second contact (34) means is arranged on the second arm segment (14).

3. The holding arm according to claim 1, wherein each contact means (30- 42) has two contact elements (30a, 30b-42a, 42b) arranged substantially opposite one another on the segment (10-22).

4. The holding arm according to claim 2 or 3, wherein the contact elements (30a, 30b-42a, 42b) are provided in the form of push buttons or touch-sensitive sensors.

5. The holding arm according to any one of the preceding claims, wherein the operating unit (28) is designed to release the associated joint (11-23) depending on the intensity of contact.

6. The holding arm according to any one of the preceding claims, wherein the operating unit (28), upon contact between the operator and both the first and the second arm segment (12, 14), releases all the joints (13, 15) disposed between said arm segments.

7. The holding arm according to any of the preceding claims, wherein the joints (11-23) comprise breaks, by means of which the joints (11-23) are releasable and lockable, wherein the brakes are preferably configured as electromagnetic breaks and comprise a permanent magnet each, which biases the break into the locked state in the unpowered state.

8. The holding arm according to any of the preceding claims, with a first interface (6) at the proximal end (2) for connecting the holding arm (1) to an energy source and to an external control unit (206) for transmitting signals to and from the holding arm (1);
a second mechatronic interface (8) at the distal end (4) for coupling the holding arm (1) to the assistance system (200) for controlling the assistance system (200); and
a transmission unit (76, 78) which is arranged inside the holding arm (1) and which connects the first interface (6) to the second interface (8) in order to transmit energy and signals between the interfaces (6, 8).

9. The holding arm according to claim 8, wherein the transmission unit comprises a bus system (76, 78).

10. The holding arm according to any of the preceding claims, wherein at least one arm segment comprises a sensor for detecting a position of the arm segment.

11. The holding arm according to claim 8, comprising a recognition unit for recognizing an assistance system coupled to the second interface, wherein the operating unit (28) is adapted to release or to lock the joints (11, 13, 15, 17, 19, 21, 23) according to the assistance system (200) coupled to the second interface (8).

12. The holding arm according to claim 8, comprising a camera which is preferably disposed at the distal end (4), wherein the camera is provided to observe an operating area and is coupled to the first interface (6) to transfer image data at the first interface (6).

13. The holding arm according to claim 8, wherein at the second interface (8) a safety element (240a, 240b) is provided, which is coupled to the operating unit (28) in such a way that the operating unit (28) locks all the joints (11, 13, 15, 17, 19, 21, 23) when the safety element (240a, 240b) indicates a faulty link between the assistance system (200) and the second interface (8).

## Revendications

1. Bras de support (1) à usage médical, en particulier pour supporter des systèmes d'assistance mécatroniques chirurgicaux et/ou des instruments chirurgicaux, comprenant
une extrémité proximale (2) pour fixer le bras de support (1) à une base et une extrémité distale (4) pour recevoir un système d'assistance mécatronique chirurgical et/ou un instrument chirurgical;
au moins un premier et un deuxième segments de bras (12, 14), le premier segment de bras (12) étant relié à une première articulation (13) et le deuxième segment de bras (14) étant relié à une deuxième articulation (15), chaque articulation (13, 15) pouvant être libérée et bloquée; et
un dispositif de commande (28) pour amener le bras de support (1) dans une pose souhaitée,
**caractérisé en ce que** le dispositif de commande (28) est conçu pour libérer l'articulation (13, 15) associée en cas de contact entre un opérateur et l'un des premier et deuxième segments de bras (12, 14), de sorte que la première articulation (13) est libérée en cas de contact entre un opérateur et le premier segment de bras (12) et la deuxième articulation (15) est libérée en cas de contact entre un opérateur et le deuxième segment de bras (14).

2. Bras de support selon la revendication 1, le dispositif de commande (28) présentant des moyens de contact (30-42) qui sont prévus pour qu'un opérateur vienne en contact avec ceux-ci, un premier moyen de contact (32) du dispositif de commande (28) étant disposé sur le premier segment de bras (12) et un deuxième moyen de contact (34) étant disposé sur le deuxième segment de bras (14).

3. Bras de support selon la revendication 2, chaque moyen de contact (30-42) présentant deux éléments de moyen de contact (30a, 30b-42a, 42b) disposés sensiblement l'un en face de l'autre sur le segment de bras (10-22).

4. Bras de support selon la revendication 2 ou 3, les éléments centraux de contact (30a, 30b-42a, 42b) étant réalisés sous la forme de palpeurs ou de capteurs tactiles.

5. Bras de support selon l'une des revendications précédentes, le dispositif de commande (28) étant configuré pour libérer l'articulation (11-23) associée en fonction de l'intensité du contact.

6. Bras de support selon l'une des revendications précédentes, le dispositif de commande (28) libérant toutes les articulations (13, 15) disposées entre ces segments de bras en cas de contact entre l'opérateur et à la fois le premier et le deuxième segment de bras (12, 14).

7. Bras de support selon l'une des revendications précédentes, les articulations (11-23) présentant des freins au moyen desquels les articulations (11-23) peuvent être libérées et bloquées, les freins étant de préférence réalisés sous la forme de freins électromagnétiques et présentant chacun un aimant permanent qui précontraint le frein dans l'état bloqué à l'état non alimenté.

8. Bras de support selon l'une des revendications précédentes, comprenant une première interface (6) à l'extrémité proximale (2) pour connecter le bras de support (1) à une source d'énergie ainsi qu'à une unité de commande externe (206) pour transmettre des signaux vers et depuis le bras de support (1);
une deuxième interface (8) à l'extrémité distale (4) pour coupler le bras de support (1) au système d'assistance (200) afin de commander le système d'assistance (200) ; et
un dispositif de transmission (76, 78) qui est disposé à l'intérieur du bras de support (1) et qui relie la première interface (6) à la deuxième interface (8) pour transmettre de l'énergie et des signaux entre les interfaces (6, 8).

9. Bras de support selon la revendication 8, le dispositif de transmission présentant un système de bus (76, 78).

10. Bras de support selon l'une des revendications précédentes, au moins un segment de bras présentant un capteur pour détecter une position du segment de bras.

11. Bras de support selon la revendication 8, comprenant une unité de reconnaissance pour reconnaître un système d'assistance couplé à la deuxième interface, le dispositif de commande (28) étant adapté pour libérer ou bloquer les articulations (11, 13, 15, 17, 19, 21, 23) en fonction du système d'assistance (200) couplé à la deuxième interface (8).

12. Bras de support selon la revendication 8, comprenant une caméra qui est de préférence disposée à l'extrémité distale (4), la caméra étant prévue pour observer un champ opératoire et étant couplée à la première interface (6) pour transmettre des données d'image à la première interface (6).

13. Bras de support selon la revendication 8, un élément de sécurité (240a, 240b) étant prévu au niveau de la deuxième interface (8), lequel est couplé au dispositif de commande (28) de sorte que le dispositif de commande (28) bloque toutes les articulations (11, 13, 15, 17, 19, 21, 23) lorsque l'élément de sécurité (240a, 240b) indique un couplage erroné entre le système d'assistance (200) et la deuxième interface (8).
